# EUROPÄISCHE PATENTSCHRIFT

(11) **EP 1 831 246 B1**
(45) Veröffentlichungstag und Bekanntmachung des Hinweises auf die Patenterteilung: **07.05.2008**
(21) Anmeldenummer: 05818326.0
(22) Anmeldetag: 13.12.2005
(51) Int. Cl.: C07K 14/00, G01N 33/68

(54) **SONDE ZUM NACHWEIS VON NUKLEINSÄUREN**
PROBE FOR DETECTING NUCLEIC ACIDS
SONDE POUR DETECTER LA PRESENCE D'ACIDES NUCLEIQUES

(30) Priorität: 23.12.2004 DE 102004063339
(43) Veröffentlichungstag der Anmeldung: 12.09.2007
(73) Patentinhaber: Bayer Technology Services GmbH, 51368 Leverkusen (DE)
(72) Erfinder: BURMEISTER, Jens, 51061 Köln (DE); SEITZ, Oliver, 10405 Berlin (DE); KÖHLER, Olaf, 79168 Rheinfelden (DE); RÖGLIN, Lars, 10245 Berlin (DE)
(86) Internationale Anmeldenummer: PCT/EP2005/013338
(87) Internationale Veröffentlichungsnummer: WO 2006/072368

(56) Entgegenhaltungen:
- O KÖHLER & O SEITZ: "Thiazole orange as fluorescent universal base in peptide nucleic acids" CHEMICAL COMMUNICATIONS - CHEMCOM., Bd. ., 2003, Seiten 2938-2939, XP002396913 GB ROYAL SOCIETY OF CHEMISTRY. in der Anmeldung erwähnt
- N SVANVIK ET AL.: "Light-up probes: thiazole orange-conjugated peptide nucleic acid for detection of target nucleic acid in homogeneous solution" ANALYTICAL BIOCHEMISTRY., Bd. 281, 2000, Seiten 26-35, XP002396911 US ACADEMIC PRESS INC. NEW YORK.

## Beschreibung

Die Erfindung betrifft eine Sonde zum Nachweis von Nukleinsäuren, Verfahren zur Herstellung der Sonde, Methoden zur Durchführung von Nachweisreaktionen sowie Testkits, die diejenigen Reagenzien enthalten, die zur Durchführung der Sonden-basierten Nachweisreaktion benötigt werden.

Der Nachweis von Nukleinsäuren hat ein großes Anwendungsgebiet z.B. in der Human- oder Veterinärdiagnostik, im Nahrungsmittelbereich, in der Umweltanalytik, im Pflanzenschutz, in der biochemischen bzw. pharmakologischen Forschung sowie in der forensischen Medizin.

Nach dem Stand der Technik erfolgt der Nachweis von Nukleinsäuren entweder durch heterogene oder homogene Assays. Heterogene Assays sind solche Methoden, bei denen mindestens ein Waschschritt benötigt wird, um gebundene und nicht gebundene Sonden voneinander zu separieren. Heterogene Assays werden beispielsweise durchgeführt, indem die Sonde an einer festen Phase immobilisiert ist, während sich die nachzuweisende Nukleinsäure in Lösung befindet. Beispiele für den Nachweis von Nukleinsäuren durch heterogene Assays sind Hybridisierungen an Filtern oder DNA Microarrays (siehe z.B. M.L.M. Anderson, Nucleic Acid Hybridization, Springer-Verlag, New York, 1998 oder D. Bowtell, J. Sambrook, DNA Microarrays, Cold Spring Harbor Laboratory Press, New York, 2003). Vorteil heterogener Assays ist die intrinsische Fähigkeit zum gleichzeitigen Nachweis mehrerer Analyten (Multiplexing), nachteilig ist zum Beispiel die Notwendigkeit von Waschschritten. Homogene Assays zeichnen sich dadurch aus, dass alle Komponenten in Lösung miteinander reagieren, wobei Waschschritte völlig entfallen. Homogene Assays sind in der Regel durch apparativ vergleichsweise einfache Methoden auslesbar, die Fähigkeit zum Multiplexing ist jedoch in der Regel begrenzt. Der Verzicht auf Waschschritte erleichtert die Automatisierung, vermindert die Gefahr von Kontaminationen und erlaubt eine kostengünstige Durchführung der Nachweisreaktionen.

Sonden zum Nachweis von DNA bestehen in der Regel aus einer hybridisierenden und einer signalgebenden Einheit. Die hybridisierende Einheit bindet sequenzspezifisch an das DNA-Target und besteht zum Beispiel aus DNA, PNA oder anderen DNA-Analoga. Die signalgebende Einheit kann zum Beispiel eine radioaktive Markierung, ein Mikro- oder Nanopartikel, ein redoxaktives Molekül, ein lumineszentes oder ein fluoreszentes Molekül sein. Die signalgebende Einheit wird in der Regel kovalent mit der hybridisierenden Einheit verknüpft. Üblicherweise erfolgt die Verknüpfung der hybridisierenden Einheit mit der signalgebenden Einheit am 5'- oder 3'-Terminus der Sonde, um eine Störung der Hybridisierung durch die signalgebende Einheit zu verhindern. Während in heterogenen Assays die Trennung von an die nachzuweisende Nukleinsäure gebundenen und frei in Lösung befindlichen Sonden durch Waschschritte erfolgt, muss in homogenen Assays sichergestellt werden, dass die signalgebende Einheit im hybridisierten Zustand der Sonde über andere Eigenschaften verfügt, als im freien Zustand. Eine Veränderung der signalgebenden Einheiten infolge der Hybridisierung der Sonde kann zum Beispiel durch Fluoreszenzresonanzenergietransfer (FRET) oder durch DNA-interkalierende Moleküle erreicht werden. Beispiele für fluoreszente, homogene Sonden, die nach dem FRET-Prinzip funktionieren, sind TaqMan^{®} Sonden (P.M. Holland et al., Proc. Natl. Acad. USA, 1991, 88, 7276-7280) oder sogenannte "Molecular Beacons" (WO 95/13399 A1; S. Tyagi und F. Kramer, Nature Biotechnol. 1996, 14, 303-307). Nach dem FRET-Prinzip funktionierende Sonden enthalten terminal einen Fluorophor und einen Quencher, wodurch die Fluoreszenz abhängig vom Hybridisierungszustand gleichsam geschaltet werden kann. Im Falle der Molecular Beacons wird als Sonde eine Haarnadel-formige DNA-Struktur verwendet, die terminal mit einem Fluorophor und einem Quencher verknüpft ist. Im nicht an das Target hybridisierten Zustand wird die Fluoreszenz durch den in räumlicher Nähe befindlichen Quencher unterdrückt. Im Falle der Target-Bindung werden Fluorophor und Quencher räumlich separiert, wodurch ein Fluoreszenzsignal generiert wird, dessen Intensität mit steigender Target-Menge zunimmt. Molecular Beacons können als Sonden in einer PCR-Reaktion eingesetzt werden, um die nach jedem Zyklus entstandene Produktmenge zu quantifizieren. Die quantitative PCR (qPCR) ist gemäß dem Stand der Technik eine der gebräuchlichsten Methoden zum Nachweis und zur Quantifizierung von Nukleinsäuren im Forschungslabor und in der Diagnostik. Nachteile der Molecular Beacons sind ihre, die Empfindlichkeit einschränkende, Eigenfluoreszenz, die begrenzte Spezifität in der Diskriminierung zwischen komplementären und einzelbasenfehlgepaarten Targets und das sehr schmale Temperaturfenster, innerhalb dessen die Sonden einsetzbar sind. Die Verknüpfung von Spezifität und Sensitivität ist die zentrale Herausforderung in der Entwicklung von Hybridisierungssonden, die insbesondere beim Nachweis von Einzelbasenmutationen (SNP) von entscheidender Bedeutung ist.

Werden interkalierende Farbstoffe, wie zum Beispiel SybrGreen, in die PCR-Mischung gegeben, so interkaliert dieser Farbstoff in den gebildeten DNA-Doppelstrang und führt zur Zunahme der Fluoreszenz mit zunehmender Produktmenge. Diese Methode ermöglicht die sondenfreie Durchführung einer quantitativen PCR (ein Beispiel findet sich in A.K. Bhar et al. J. Clin. Microbiol. 2001, 39, 2835-2845). Nachteil der Methode ist jedoch die Eigenfluoreszenz des Farbstoffes, der in hoher Konzentration eingesetzt werden muss, sowie die völlig unspezifische Anfärbung. Auch ein Multiplexing der qPCR ist mit diesem Verfahren nur begrenzt erreichbar und gelingt zum Beispiel durch die Aufnahme von Schmelzkurven, durch die sich verschiedene Produkte diskriminieren lassen. DNA-bindende, signalgebende Moleküle, deren Signaleigenschaften sich durch die DNA-Bindung ändern, können ebenfalls für die Entwicklung homogener Sonden genutzt werden, wobei durch die Verknüpfung des signalgebenden Moleküls mit der Sonde die Spezifität sichergestellt wird. Dieses Prinzip wurde zuerst von Barton et al. realisiert, die Ruthenium(II)-Komplexe mit DNA-Sonden verknüpften (US 5,157,032; Y. Jenkins et al., Biochemistry 1992, 31, 10809-10816). Diese Sonden zeigen einen Fluoreszenzanstieg bei der Hybridisierung. Jedoch ist bei Zugabe von doppelsträngiger Nicht-Target-DNA ebenfalls ein Fluoreszenzanstieg zu verzeichnen. Der kationische Ruthenium-Komplex begünstigt zudem unspezifische Hybridisierungen und schränkt die Spezifität der Sonden signifikant ein. In EP 0 710 668 B 1 wird der Nachweis von Nukleinsäuren durch DNA-Sonden beschrieben, die terminal mit einem asymmetrischen Cyanin-Farbstoff verknüpft sind. Durch die Hybridisierung dieser Sonden wird ein schwacher Fluoreszenzanstieg, etwa um den Faktor 4 erreicht, der keinen empfindlichen Nukleinsäure-Nachweis ermöglicht. In US 6,329,144 B1 wird eine PNA-Sonde beschrieben, die terminal mit einem asymmetrischen Cyanin-Farbstoff verknüpft ist. Diese sogenannte LightUp^{®}-Sonde zeigt einen deutlichen Fluoreszenzanstieg infolge der Hybridisierung an Nukleinsäuren. Das Kriterium der Sensitivität wird durch diese Sonden erfüllt und konnte auch im Kontext der qPCR demonstriert werden (N. Svanvik et al., Anal. Biochemistry 2000, 287, 179-182). Hinsichtlich der Spezifität sind die LightUp^{®}-Sonden jedoch auf die Diskriminierungsfähigkeit der PNA-Sequenz selbst beschränkt, weshalb beim Nachweis von Einzelbasenmutationen nur ein begrenzter Unterschied der Fluoreszenzintensitäten zwischen passenden und nichtpassenden Basenpaarungen erreicht werden kann (Beispiel ist die schwierige Unterscheidung von G:C und G:T Basenpaaren).

Privat et al. beschrieben DNA-Sonden, in denen eine zentrale, internukleotidische Phosphatgruppe über einen Linker mit Thiazolorange verknüpft wurde (E. Privat et al., Photochem. Photobiol. 2002, 75, 201-210). Diese Sonden zeigten nur einen geringen Fluoreszenzanstieg um den Faktor fünf bei der Hybridisierung an komplementäre Targets. Aufgrund der resultierenden, geringen Nachweisempfindlichkeit sind die beschriebenen Sonden nicht für die praktische Anwendung in der Nukleinsäure-Diagnostik geeignet.

Eine DNA-Sonde zum homogenen Nachweis von Nukleinsäuren auf der Basis fluoreszenter Nukleoside wurde von Okamoto et al. beschrieben (A. Okamoto et al., J. Am. Chem. Soc. 2003, 125, 9296-9297). Als fluoreszente Nukleoside wurden Methoxybenzodeazaadenin (^{MD}A) und Methoxybenzodeazainosin (^{MD}I) in die Sonden eingebaut, wobei ^{MD}A bei Anwesenheit von C im Gegenstrang deutlich fluoresziert, während ^{MD}I in Anwesenheit von T im Gegenstrang deutlich fluoresziert. Diese Sonden sind prinzipiell zur Diskriminierung von Einzelbasenmutationen in homogenen Assays geeignet, nachteilig ist jedoch die Tatsache, dass die Fluoreszenz der fluoreszenten Nukleoside durch flankierende G:C Basenpaare unterdrückt wird (Fluoreszenz-Quenching). Diese Eigenschaft schränkt die generelle Anwendbarkeit dieser Sonden empfindlich ein. Ein ähnliches Prinzip wird in WO2004058793 A1 offenbart: Die Nukleobasen Uracil und Cytosin wurden über einen Propargyl-Linker mit Pyrencarboxamid verknüpft und die so erhaltenen fluoreszenten Basen (^{Py}U und ^{Py}C) in DNA-Sonden integriert. Zwar zeigen die passenden Basenpaarungen ^{Py}U:A und ^{Py}C:G eine deutlich höhere Fluoreszenz als die schwächer paarenden Kombinationen, allerdings führt die Hybridisierung der ^{Py}U- und ^{Py}C-Sonden nur zu geringen Fluoreszenzanstiegen (2-8fach). Ein weiterer Nachteil dieses Verfahrens ist, dass nur ein Fluoreszenzfarbstoff zur Verfügung steht und somit kein Multiplexing des Assays realisiert werden kann.

Köhler und Seitz zeigten, dass der Fluoreszenzfarbstoff Thiazol-Orange als universelles Basensurrogat an eine PNA-Sonde mit Aminoethylglycin-Rückgrat angebunden werden kann und so den Aufbau einer PNA-Sonde mit verbesserter Spezifität ermöglicht (O. Köhler, O. Seitz, Chem. Commun. 2003, 23, 2938-2939). Werden mit diesen Sonden Einzelbasenmutationen nachgewiesen, so wird die universelle Base Thiazolorange neben der Mutationsstelle eingeführt. Der umgebungssensitive Farbstoff ergibt nur dann ein maximales Fluoreszenzsignal, wenn er sich in Nachbarschaft perfekter Basenpaarungen befindet. Experimente mit dieser Sonde zeigten, dass der perfekt gepaarte Duplex zwischen der Sonde und einem dazu komplementären Target selbst dann noch mehr Fluoreszenz als der eine Fehlpaarung enthaltende Duplex zeigte, wenn die Hybridisierung unterhalb des Duplex-Schmelzpunktes stattfand. Diese Beobachtung zeigt, dass mittels Thiazolorange eine Spezifität der Sonden erreichbar ist, die über die Spezifität der komplementären Basenpaarung hinausgeht. Während die Spezifität dieser Sonden auf der Basis einer PNA-Sequenz mit Aminoethylglycin-Rückgrat eine Verbesserung gegenüber dem Stand der Technik darstellt, ist der erzielte Faktor der Fluoreszenzverstärkung mit anderen homogenen Sonden vergleichbar.

Die Verbesserung von Spezifität und Sensitivität ist die zentrale Aufgabenstellung in der Entwicklung von Hybridisierungssonden, die insbesondere beim Nachweis von Einzelbasenmutationen (SNP) von entscheidender Bedeutung ist. Zusätzlich sollten die Sonden über einen möglichst breiten Temperaturbereich einsetzbar sein und das Multiplexing der Assays ermöglichen.

Der vorliegenden Erfindung liegt daher die Aufgabe zugrunde, eine Sonde zum Nachweis von Nukleinsäuren bereitzustellen, die mit hoher Spezifität und Selektivität sowie über einen möglichst großen Temperaturbereich hybridisiert, in homogenen Assays einsetzbar ist und Multiplex-Nachweise erlaubt.

Es wurde nun überraschenderweise gefunden dass es eine Sonde gibt, die diese Aufgabe löst.

Gegenstand der Erfindung ist daher eine Sonde zum Nachweis von Nukleinsäuren, die aus einem Peptidnukleinsäurestrang, einem Nukleinsäurestrang oder einem Strang aus DNA-Analoga besteht, in dem eine Nukleobase an interner Position durch ein fluoreszentes Basensurrogat ersetzt wurde. Überraschend zeigte sich, dass beispielsweise Thiazolorange, das an Ornithin gebunden wurde, bei der Hybridisierung an eine Target-Sequenz ohne Fehlpaarung eine deutlich höhere Fluoreszenzverstärkung zeigte, als Thiazolorange, das nach dem Stand der Technik an ein Aminoethylglycin-Rückgrat angebunden wurde. Eine weiterhin überraschende Beobachtung war, dass L-Ornithin- und D-Ornithin-Konjugate sich hinsichtlich des Verhältnisses der Spezifität der Hybridisierung zu der erreichbaren Fluoreszenzverstärkung unterscheiden: Das L-Ornithin-Konjugat zeigte eine höhere Fluoreszenzverstärkung als das D-Omithin-Konjugat, während das D-Ornithin-Konjugat eine höhere Spezifität der Fluoreszenzverstärkung zeigte.

Gegenstand der Erfindung ist daher eine durch Festphasen-Synthese erhältliche PNA-Sonde der allgemeinen Formel: 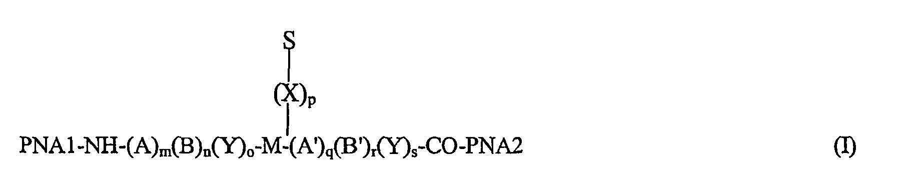 in der

PNA1 ein Peptidnukleinsäurestrang beliebiger Sequenz und Länge, bevorzugt eine Sequenz von 2-100 Basen, besonders bevorzugt zwischen 2-10 Basen ist.

PNA2 ein Peptidnukleinsäurestrang beliebiger Sequenz und Länge, bevorzugt eine Sequenz von 2-100 Basen, besonders bevorzugt zwischen 2-10 Basen ist.

A und A' unabhängig voneinander für eine Methylengruppe oder eine substituierte Kohlenstoffeinheit des Typs CHR, oder CR2 steht, wobei R für Wasserstoff oder einen organischen Rest, bevorzugt eine substituierte oder unsubstituierte verzweigte oder unverzweigte C1-C10-Alkylgruppe - wobei die Substituenten bevorzugt aus der Gruppe substituierter oder unsubstituierter Aminogruppen, Elementen der 5. oder 6. Hauptgruppe, bevorzugt O oder S, gewählt sein können -, eine substituierte oder unsubstituierte Phenylgruppe, besonders bevorzugt für die jeweils unsubstituierten und unverzweigten Ausführungsformen und ganz besonders bevorzugt für Wasserstoff oder eine Methylgruppe steht, bedeutet, bevorzugt steht A für eine Methylengruppe.

B und B' unabhängig voneinander für eine Methylengruppe oder eine substituierte Kohlenstoffeinheit des Typs CHR, oder CR2 steht, wobei R für Wasserstoff oder einen organischen Rest, bevorzugt eine substituierte oder unsubstituierte verzweigte oder unverzweigte C1-C10-Alkylgruppe - wobei die Substituenten bevorzugt aus der Gruppe substituierter oder unsubstituierter Aminogruppen, Elementen der 5. oder 6. Hauptgruppe, bevorzugt O oder S, gewählt sein können -, eine substituierte oder unsubstituierte Phenylgruppe, besonders bevorzugt für die jeweils unsubstituierten und unverzweigten Ausführungsformen und ganz besonders bevorzugt für Wasserstoff oder eine Methylgruppe steht, bedeutet, bevorzugt steht B für eine Methylengruppe.
m eine natürliche Zahl von 1-5, bevorzugt 1 bedeutet,
n eine natürliche Zahl von 1-5, bevorzugt 1 bedeutet,
o eine natürliche Zahl von 1-5, bevorzugt 1 bedeutet,

X für eine Methylengruppe oder eine substituierte Kohlenstoffeinheit des Typs CHR, oder CR2 steht, wobei R für Wasserstoff oder einen organischen Rest, bevorzugt eine substituierte oder unsubstituierte verzweigte oder unverzweigte C1-C10-Alkylgruppe - wobei die Substituenten bevorzugt aus der Gruppe substituierter oder unsubstituierter Aminogruppen, Elementen der 5. oder 6. Hauptgruppe, bevorzugt O oder S, gewählt sein können -, oder eine substituierte oder unsubstituierte Phenylgruppe steht, oder

X für eine substituierte oder unsubstituierte Aminogruppe des Typs NH oder NR wobei R für Wasserstoff oder einen organischen Rest, bevorzugt eine substituierte oder unsubstituierte verzweigte oder unverzweigte C1-C10-Alkylgruppe - wobei die Substituenten bevorzugt aus der Gruppe, Elementen der 5. oder 6. Hauptgruppe, bevorzugt O oder S, gewählt sein können -, eine substituierte oder unsubstituierte Phenylgruppe steht,
oder X für ein Atom der 5. oder 6. Hauptgruppe des Periodensystems, bevorzugt Sauerstoff oder Schwefel steht,
besonders bevorzugt steht X für eine NH-Gruppe.
p eine natürliche Zahl von 0 bis 10, bevorzugt eine Zahl von 1-6 ist,
S ein fluoreszentes, universelles Basensurrogat, dadurch gekennzeichnet, dass es fluoreszent und zur Interkalation in DNA befähigt ist, bevorzugt ein Cyaninfarbstoff, weiterhin bevorzugt Ethidium, weiterhin bevorzugt ein substituiertes Anthracen, weiterhin bevorzugt ein Acridin-Farbstoff, weiterhin bevorzugt ein Farbstoff ausgewählt aus den von der Firma Molecular Probes vertriebenen Farbstoffen PO-PRO-1, BO-PRO-1, YO-PRO-1, TO-PRO-1, JO-PRO-1, PO-PRO-1, PO-PRO-3, LO-PRO-1, BO-PRO-3, YO-PRO-3, TO-PRO-3, TO-PRO-5 (sowie von diesen Verbindungen abgeleitete Fluorophore mit veränderter Linkerstruktur), besonders bevorzugt Thiazolorange ist,
M für eine substituierte Kohlenstoffeinheit des Typs CH, oder CR (wobei R Wasserstoff steht, wobei R für Wasserstoff oder einen organischen Rest, bevorzugt eine substituierte oder unsubstituierte verzweigte oder unverzweigte C1-C10-Alkylgruppe - wobei die Substituenten bevorzugt aus der Gruppe substituierter oder unsubstituierter Aminogruppen, Elementen der 5. oder 6. Hauptgruppe, bevorzugt O oder S, gewählt sein können -, oder eine substituierte oder unsubstituierte Phenylgruppe steht, oder
M für eine substituierte oder unsubstituierte Aminogruppe des Typs NH oder NR wobei R für Wasserstoff oder einen organischen Rest, bevorzugt eine substituierte oder unsubstituierte verzweigte oder unverzweigte C1-C10-Alkylgruppe - wobei die Substituenten bevorzugt aus der Gruppe, Elementen der 5. oder 6. Hauptgruppe, bevorzugt O oder S, gewählt sein können -, eine substituierte oder unsubstituierte Phenylgruppe steht,
oder M für ein Atom der 5. Oder 6. Hauptgruppe des Periodensystems, bevorzugt Sauerstoff oder Schwefel steht,
besonders bevorzugt steht M für eine CH-Gruppe.

Y wie A definiert ist.
q eine natürliche Zahl von 0-5, bevorzugt 0 ist,
r eine natürliche Zahl von 0-5, bevorzugt 0 ist,
s eine natürliche Zahl von 0-5, bevorzugt 0 ist.

Das hybridisierende Oligomer vom Nucleinsäuretyp kann alternativ zu PNA auch DNA oder ein DNA-Analogon wie beispielsweise LNA, Morpholino-DNA oder Arabino-DNA sein. Dementsprechend sind auch durch Festphasen-Synthese erhältliche Sonden der allgemeinen Formel (II) Gegenstand der Erfindung: 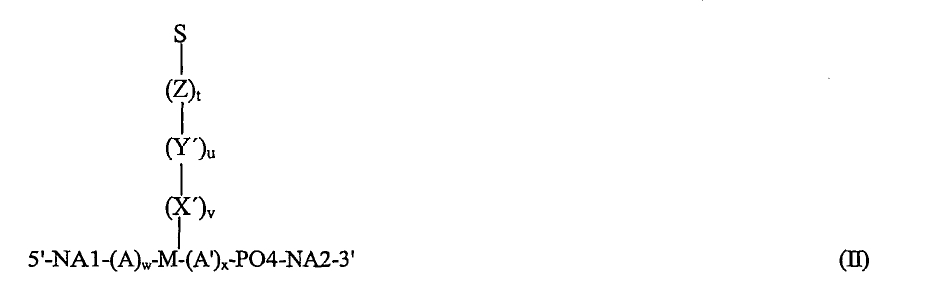 in der
5'-NA1 eine Nukleinsäure oder ein Nukleinsäureanalogon beliebiger Sequenz und Länge, die mit einer 3'-terminalen Phosphatgruppe endet, bevorzugt eine Nukleinsäure-Sequenz von 3-100 Basen Länge, besonders bevorzugt eine Nukleinsäure-Sequenz von 3-20 Basen Länge, ganz besonders bevorzugt eine Nukleinsäure-Sequenz von 3-15 Basen Länge ist,
NA2-3' eine Nukleinsäure oder ein Nukleinsäureanalogon beliebiger Sequenz und Länge, bevorzugt eine Nukleinsäure-Sequenz von 3-100 Basen Länge, besonders bevorzugt eine Nukleinsäure-Sequenz von 3-20 Basen Länge, ganz besonders bevorzugt eine Nukleinsäure-Sequenz von 3-15 Basen Länge ist,
A und A' wie oben definiert ist.
w und x eine natürliche Zahl von 0-5 ist,
X' und Z unabhängig voneinander für eine Methylengruppe oder eine substituierte Kohlenstoffeinheit des Typs CHR, oder CR2 steht, wobei R für Wasserstoff oder einen organischen Rest, bevorzugt eine substituierte oder unsubstituierte verzweigte oder unverzweigte C1-C10-Alkylgruppe - wobei die Substituenten bevorzugt aus der Gruppe substituierter oder unsubstituierter Aminogruppen, Elementen der 5. oder 6. Hauptgruppe, bevorzugt O oder S, gewählt sein können -, oder eine substituierte oder unsubstituierte Phenylgruppe steht, oder
X' und Z unabhängig voneinander für eine substituierte oder unsubstituierte Aminogruppe des Typs NH oder NR wobei R für Wasserstoff oder einen organischen Rest, bevorzugt eine substituierte oder unsubstituierte verzweigte oder unverzweigte C1-C10-Alkylgruppe - wobei die Substituenten bevorzugt aus der Gruppe, Elementen der 5. oder 6. Hauptgruppe, bevorzugt O oder S, gewählt sein können -, eine substituierte oder unsubstituierte Phenylgruppe steht, oder
X' und Z unabhängig voneinander für ein Atom der 5. oder 6. Hauptgruppe des Periodensystems, bevorzugt Sauerstoff oder Schwefel steht,
besonders bevorzugt stehen X' und Z unabhängig voneinander für eine Methylengruppe.
Y' für eine Methylengruppe oder eine substituierte Kohlenstoffeinheit des Typs CHR, oder CR2 steht, wobei R für Wasserstoff oder einen organischen Rest, bevorzugt eine substituierte oder unsubstituierte verzweigte oder unverzweigte C1-C10-Alkylgruppe - wobei die Substituenten bevorzugt aus der Gruppe substituierter oder unsubstituierter Aminogruppen, Elementen der 5. oder 6. Hauptgruppe, bevorzugt O oder S, gewählt sein können -, oder eine substituierte oder unsubstituierte Phenylgruppe steht, oder
Y' für eine substituierte oder unsubstituierte Aminogruppe des Typs NH oder NR wobei R für Wasserstoff oder einen organischen Rest, bevorzugt eine substituierte oder unsubstituierte verzweigte oder unverzweigte C1-C10-Alkylgruppe - wobei die Substituenten bevorzugt aus der Gruppe, Elementen der 5. oder 6. Hauptgruppe, bevorzugt O oder S, gewählt sein können - eine substituierte oder unsubstituierte Phenylgruppe steht,
oder Y' für ein Atom der 5. Oder 6. Hauptgruppe des Periodensystems, bevorzugt Sauerstoff oder Schwefel steht,
besonders bevorzugt steht Y' für eine NH-Gruppe.
v eine natürliche Zahl von 0 bis 10, bevorzugt eine Zahl von 1-6, besonders bevorzugt 1 ist,
u eine natürliche Zahl von 0 bis 10, bevorzugt eine Zahl von 1-6, besonders bevorzugt 1 ist,
t eine natürliche Zahl von 0 bis 10, bevorzugt eine Zahl von 1-6 ist,

S und M wie oben definiert sind.

Bevorzugt, besonders bevorzugt oder ganz besonders bevorzugt sind Ausführungsformen, welche von den unter bevorzugt, besonders bevorzugt oder ganz besonders bevorzugt genannten Parametern, Verbindungen, Definitionen und Erläuterungen Gebrauch machen.

Die in der Beschreibung aufgeführten allgemeinen oder in Vorzugsbereichen aufgeführten Definitionen, Parameter, Verbindungen und Erläuterungen können jedoch auch untereinander, also zwischen den jeweiligen Bereichen und Vorzugsbereichen beliebig kombiniert werden.

Das erfindungsgemäße, fluoreszente, universelle Basensurrogat S, wie in den Sonden gemäß Formel (I) und (II) erwähnt, ist dadurch gekennzeichnet, dass es zur Interkalation in DNA befähigt ist und sich unabhängig von der im Nukleinsäure-Target dem Surrogat gegenüber liegenden Base die gleiche oder eine ähnliche Schmelztemperatur für den DNA-Sonden-Duplex ergibt. Insofern kann das Basensurrogat als universell bezeichnet werden. Beispiele für erfindungsgemäße Basensurrogate sind Cyanin-Farbstoffe wie beispielsweise Thiazolorange, Oxazolgelb (EP 0 714 986 Al), Ethidium, Dapoxyl^{®}, 2,6-Donor-Acceptor-substituierte Anthracene (H. Ihmels et al., Org. Lett. 2000, 2, 2865-2867), Acridine (siehe zum Beispiel K. Fukui et al., Bioconjugate Chem. 1996, 7, 349-355), von der Firma Molecular Probes vertriebene Farbstoffe (siehe Handbuch der Firma Molecular Probes: Handbook of Fluorescent Probes and Research Products) wie zum Beispiel PO-PRO-1, BO-PRO-1, YO-PRO-1, TO-PRO-1, JO-PRO-1, PO-PRO-1, PO-PRO-3, LO-PRO-1, BO-PRO-3, YO-PRO-3, TO-PRO-3, TO-PRO-5 (sowie von diesen Fluorophoren abgeleitete Verbindungen mit veränderter oder fehlender Linkerstruktur) und der BTCS-Chromophor (der BTCS Chromophor wird beispielsweise beschrieben in R. B. Thompson et al., J. Biomed. Opt. 2000, 5, 1, 17-22). Sonden, die sich in ihrer Sequenz unterscheiden, können durch Verwendung von Basensurrogaten, die sich hinsichtlich ihrer Fluoreszenzanregungs- und Fluoreszenzemissionswellenlängen unterscheiden gleichsam farbig markiert werden und ermöglichen so den gleichzeitigen Nachweis mehrerer Analyten (Multiplexing).

Das fluoreszente Basensurrogat ist an ein Rückgrat angebunden, das einen längeren Abstand zwischen den das Basensurrogat flankierenden Nukleobasen herstellt, als ein den Abstand benachbarter Nukleobasen in der DNA imitierendes Rückgrat. Beispiel für ein die Standardgeometrie der DNA imitierendes Rückgrat ist das Aminoethylglycin-Rückgrat in der PNA. Je mehr der Abstand zwischen dem fluoreszenten Basensurrogat und den flankierenden Nukleobasen von der Standardgeometrie des DNA-Rückgrats abweicht, umso weniger effizient können Basenstapelwechselwirkungen die Emission des fluoreszenten Basensurrogats im Einzelstrang erhöhen. Als Resultat ist die Fluoreszenz des Sondeneinzelstrangs gering. Der Abstand darf jedoch nicht so groß gewählt werden, dass eine Basenstapelung im nach Hybridisierung gebildeten Doppelstrang verhindert würde. Ein geeigneter Abstand zwischen dem fluoreszenten Basensurrogat und den flankierenden Nukleobasen innerhalb einer PNA-Sonde mit Aminoethylglycin-Rückgrat kann beispielsweise durch Einführung von 3-10 Methylengruppen an der das Basensurrogat tragenden Rückgratposition hergestellt werden. Die das Basensurrogat tragende Rückgratstruktur kann neben Kohlenstoffeinheiten auch Heteroatome wie zum Beispiel Sauerstoff, Stickstoff, Schwefel, Phosphor oder Silicium enthalten. Ein Beispiel für ein erfindungsgemäßes Rückgrat an der Stelle des fluoreszenten Basensurrogates ist Ornithin innerhalb einer PNA-Sonde mit Aminoethylglycin-Rückgrat.

Werden mehrere Einheiten der allgemeinen Struktur (I) oder (II) kovalent miteinander verknüpft, so kann eine Sonde mit multiplen Markierungen erhalten werden. Ein solches Vorgehen steigert die Nachweisempfindlichkeit bei der Verwendung der erfindungsgemäßen Sonden. Im Gegensatz zu terminalen Fluoreszenzmarkierungen, durch die maximal zwei Markierungen pro Sonde möglich sind, wird durch Einbau der fluoreszenten Basensurrogate eine multiple Markierung möglich.

Die erfindungsgemäße Sonde eignet sich zur Durchführung von Hybridisierungsassays, insbesondere von homogenen Hybridisierungsassays. In homogenen Hybridisierungsassays kann die Sonde beispielsweise eingesetzt werden, um die Art und Menge einer in einer wässrigen Probe vorkommenden Target-Nukleinsäure zu bestimmen. Hierbei wird die Sonde zu der gelösten Target-Nukleinsäure gegeben und für eine Zeit von wenigen Sekunden bis zu mehreren Stunden inkubiert. Anschließend wird die Fluoreszenz der Lösung im Vergleich zu einer Kontrolle (gleiche Lösung ohne Target-Nukleinsäure) gemessen. Der Vergleich des Fluoreszenzwertes mit einer Standardkurve, in der die Korrelation zwischen der Fluoreszenz der Sonde und der eingesetzten Target-Menge ermittelt wurde, ermöglicht die Quantifizierung der nachzuweisenden Nukleinsäure. Soll der quantitative Nachweis von Nukleinsäuren mit besonders hoher Empfindlichkeit durchgeführt werden, so kann die einfach oder mehrfach markierte Sonde besonders vorteilhaft mit dem Verfahren der PCR kombiniert werden. Während der quantitativen PCR wird die Zunahme der Fluoreszenz in Folge der Target-Amplifikation bestimmt. Die Anzahl der PCR-Zyklen, die zur Überschreitung eines festgelegten Schwellwertes der Fluoreszenz benötigt wird, definiert man als sogenannten Ct-Wert (Cycle threshold). Der Ct-Wert kann über eine Eichkurve in Beziehung zur eingesetzten Target-Konzentration gesetzt werden und stellt somit ein Maß für die Target-Konzentration dar. Die Sonde muss dabei so gewählt werden, dass sie komplementär zu einem zwischen den Primer-Sequenzen liegenden Sequenzabschnitt des Targets ist und möglichst wenig Kreuzhybridisierung zu anderen Sequenzen des Genoms zeigt.

Die Sonde eignet sich für den Nachweis von Einzelbasenmutationen (SNP), wobei dieser Nachweis beispielsweise durch Hybridisierung oder durch PCR erfolgen kann. Auch Methylierungsmuster können mit der Sonde analysiert werden. Wird die zu untersuchende DNA mit Natriumbisulfit behandelt, so werden alle nicht methylierten Cytosine in Uracil umgewandelt, während methylierte Cytosine unverändert aus der Reaktion hervorgehen. Das resultierende, teilweise veränderte Target kann dann durch Assays wie z.B. PCR, quantitative PCR oder Hybridisierung analysiert werden. Ein solches Verfahren unter Verwendung der PCR bzw. quantitativen PCR wird zum Beispiel in J. G. Herman et al., Proc. Natl. Acad. Sci. USA 1996, 93, 9821-9826 et al., bzw. in T.-S. Wong et al., European Journal of Cancer 2003, 39, 1881-1887 beschrieben. Für die Analyse von mit Natriumbisulfit behandelter DNA können die Sonden in Assays verwendet werden, bevorzugt in der PCR oder in der quantitativen PCR.

Die Herstellung der Sonde erfolgt durch chemische Synthese, bevorzugt durch Festphasensynthese, besonders bevorzugt durch automatisierte Festphasensynthese. Methoden zur Herstellung von Nukleinsäuren, Nukleinsäure-Analoga und Peptidnukleinsäuren sind dem Fachmann bekannt und werden zum Beispiel in L.M. Smith, Anal. Chem. 1988, 60, 381-390 sowie in B. E. Hyrup und P. E: Nielsen, Bioorg. Med. Chem. 1996, 4, 5-23 beschrieben. Die Sonden können entweder im Anschluß an die Festphasensynthese kovalent mit dem fluoreszenten Basensurrogat verknüpft werden, oder der Einbau des Basensurrogates erfolgt bereits während der konvergenten Synthese. Ornithin kann beispielsweise N-terminal durch eine Fluorenylmethoxycarbonylgruppe (Fmoc-Gruppe) geschützt werden, während das Basensurrogat kovalent mit der primären Aminogruppe des Ornithins verknüpft wird. Beispielsweise kann Thiazolorange über eine Carboxylgruppe an die primäre Aminogruppe von Ornithin gebunden werden. Ein solcher Baustein eignet sich zum Einsatz in automatisierten Festphasensynthesen der Sonden nach dem Fmoc-Verfahren.

Die erfindungsgemäße Sonde besitzt gegenüber den aus dem Stand der Technik bekannten Sonden zum Nachweis von Nukleinsäuren folgende Vorteile:
a) Hohe Spezifität der Erkennung zwischen der Sonde und der nachzuweisenden Nukleinsäure, die insbesondere beim Nachweis von Einzelbasenmutationen (SNP) oder methylierten Cytosinen von Vorteil ist.
b) Geringe Fluoreszenz im Einzelstrang aufgrund der Aufweitung des Rückgrates und infolgedessen erhöhte Anstiege der Fluoreszenz bei Bildung der Sonden-Analyt-Komplexe.
c) Möglichkeit, durch Auswahl des Rückgrates entweder Sonden mit besonders hoher Fluoreszenzverstärkung (=Nachweisempfindlichkeit) oder Spezifität zu generieren.
d) Durch Einbau mehrerer Basensurrogate kann die Sonde mehrfach markiert werden.
e) Einsatz in Multiplex-Assays durch Verwendung von Basensurrogaten, deren Fluoreszenzmaxima bei verschiedenen Wellenlängen liegen.
f) Hybridisierung und Einzelbasen-spezifische Diskriminierung in einem weiten Temperaturbereich bei nicht-stringenten Hybridisierungsbedingungen. Dieses erleichtert das Sondendesign und die Optimierung der Assays erheblich.
g) Die Herstellung der Sonden erfolgt durch konventionelle Festphasen-Synthese.

Im Folgenden wird die Erfindung anhand von Ausführungsbeispielen und Abbildungen näher erläutert.

Es zeigen
Figur 1
   das Verhältnis zwischen der Fluoreszenzintensität der PNA-Sonde Ac-GCCGTA-R(TO)-TAGCCG nach und vor Zugabe des DNA-Targets 5'-CGGCTAZTACGGC-3' in Abhängigkeit vom Rückgrat R, an das Thiazolorange (TO) gebunden wurde und in Abhängigkeit des dem Basensurrogat mit Z= 1= Thymidin (T), 2= Guanosin (G), 3= Adenosin (A) und 4= Cytidin (C) im Duplex gegenüberliegenden Nukleotids. Dabei entspricht 5= R=Aminoethylglycin (Aeg), 6= R= *L-*Ornithin (L-Orn) und 7= R = *D*-Ornithin (D-Orn).
Figur 2
   das Verhältnis zwischen der Fluoreszenzintensität der PNA-Sonde Ac-GCCGTA-R(TO)-TAGCCG nach und vor Zugabe des DNA-Targets 5'-CGGCTYTTACGGC-3' in Abhängigkeit vom Rückgrat R, an das Thiazolorange (TO) gebunden wurde und in Abhängigkeit von der im Target vorkommenden Einzelbasenmutation mit Y= 8= Adenin (A), 9= Guanosin (G), 10= Cytidin (C) und 11= Thymidin (T). Dabei entspricht 5= R=Aminoethylglycin (Aeg), 6= R= *L*-Ornithin (L-Orn) und 7= R = D-Ornithin (D-Orn).

### Beispiele zur Synthese der Sonden

Die PNA-Monomere wurden von der Firma PerSeptive Biosystems bezogen und vor der Synthese im Feinvakuum getrocknet. Die Festphase NovaSyn^{®} TGR, PyBOP und D-Ornithin-hydrochlorid wurden bei Novabiochem erworben, L-Ornithin-hydrochlorid bei Avocado Research Chemicals. Die DNA-Oligonukleotide wurden von der Firma MWG-Biotech bezogen. Alle weiteren Chemikalien bezog man von den Firmen Acros, Aldrich, Avocado, Fluka und Riedel de Haen. Die verwendeten Lösungsmittel wurden vor Gebrauch ggf. destilliert bzw. nach Standardverfahren getrocknet. Alle wässrigen Lösungen wurden mit Wasser hergestellt, das mittels einer Milli-Q-Pore-Anlage (Firma Millipore) gereinigt wurde.

Die Synthese der Sonden kann durch eine divergente oder durch eine konvergente (lineare) Strategie erfolgen. Im Folgenden wird die Synthese der Sonden unter Anwendung einer linearen Synthesestrategie beschrieben, da letztere vollautomatisiert durchführbar ist. Als Beispiel für eine Ausführungsform der Erfindung synthetisierte man die Fmoc-geschützten Thiazolorange-Ornithine 1 und 2.

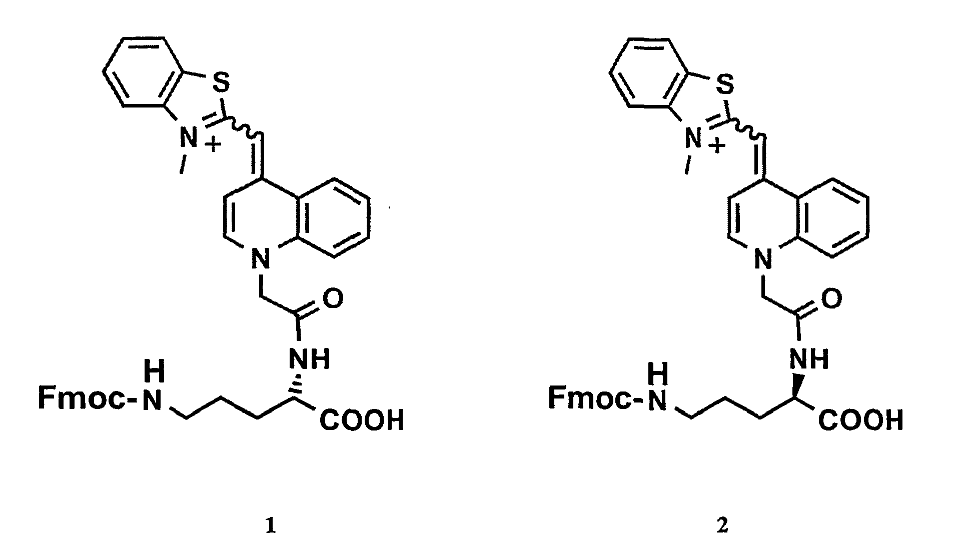

### Beispiel 1: Herstellung von Nδ-[9-(Fluorenyhnethoxycarbonyl)-L-ornithin]-Nα-[2-(1-carboxymethyl-1H-quinolin-4-ylidenmethyl)-3-methyl-benzothiazol-3-ium bromid]allylester (Fmoc-L-Orn(TO)-OAll)

Thiazolorange-Carbonsäure wurde nach einer Vorschrift von Zhou et al. hergestellt (X.-F- Zhou te 1., Journal of Imaging Science and Technology, 1995, 39, 244-252). Zu einer Lösung von Thiazolorange-Carbonsäure (100 mg, 0,23 mmol) in trockenem DMF (2,3 ml) fügte man PyBOP (145 mg, 0,279 mmol), Pyridinium-p-Toluol-Sulfonat (58 mg, 0,232 mmol) und N-Methylmorpholin (23 mg, 0,23 mmol). Die Suspension wurde unter Argon-Atmosphäre gerührt, bis die klare Lösung A erhalten wurde. L-Ornithin wurde nach Standardverfahren N-terminal mit Fmoc und C-terminal mit einer Allylgruppe geschützt (= Fmoc-L-Orn-OAll). Die klare Lösung A wurde zu einer Lösung von Fmoc-L-Orn-OAll (100 mg, 0,23 mmol) und N-Methylmorpholin (23 mg, 0,23 mmol) in DMF (2,3 ml) gegeben. Das Reaktionsgemisch wurde 12h unter Argon-Atmosphäre gerührt. Das Lösungsmittel wurde im Vakuum entfernt, den Rückstand nahm man in Methanol (2 ml) auf. Man rührte 1h, filtrierte den Feststoff ab und wusch mit Methanol nach (5 ml). Das Rohprodukt wurde durch Säulenchromatographie gereinigt (Chloroform : Methanol 97:3, 1% Ameisensäure). Man erhielt 118 mg Fmoc-L-Om(TO)-OAll in Form eines orange-farbenen Feststoffes, entsprechend einer Ausbeute von 63%.
Rf (Chloroform : Methanol 80:20, 1% Ameisensäure) = 0,56

### Beispiel 2: Herstellung von Nδ-[9-(Fluorenylmethoxycarbonyl)-L-ornithin]-Nα-[2-(1-carboxymethyl-1H-quinolin-4-ylidenmethyl)-3-methyl-benzothiazol-3-ium bromid] OH (Fmoc-L-Orn(TO)-OH) 1

Zu einer Lösung von Fmoc-L-Orn(TO)-OAll (81 mg, 0,1 mmol) in THF (5 ml) gab man N-Methylanilin (11,3 mg, 0,1 mmol) und entgaste die Lösung mehrmals. Nach Zugabe von [Pd(PPh₃)₄] (12 mg, 0,01 mmol) rührte man die Mischung 14 h unter Lichtausschluß. Das Lösungsmittel wurde im Vakuum entfernt. Den Rückstand löste man in Methanol (4ml) und filtrierte das Präzipitat ab. Das Rohprodukt wurde säulenchromatographisch gereinigt (Chloroform : Methanol 97:3, 1% Ameisensäure). Man erhielt 53 mg des Produktes Fmoc-L-Orn(TO)-OH (1) in Form eines orange-farbenen Feststoffes, entsprechend einer Ausbeute von 68%.
Rf (Chloroform : Methanol 80:20, 1 % Ameisensäure) = 0,22

### Beispiel 3: Herstellung von Nδ-[9-(Fluorenylmethoxycarbonyl)-D-ornithin]-Nα-[2-(1-carboxymethyl-1H-quinolin-4-ylidenmethyl)-3-methyl-benzothiazol-3-ium bromid]allylester (Fmoc-D-Orn(TO)-OAll)

Thiazolorange-Carbonsäure wurde nach einer Vorschrift von Zhou et al. hergestellt (X.-F- Zhou te 1., Journal of Imaging Science and Technology, 1995, 39, 244-252). Zu einer Lösung von Thiazolorange-Carbonsäure (100 mg, 0,23 mmol) in trockenem DMF (2,3 ml) fügte man PyBOP (145 mg, 0,279 mmol), Pyridinium-p-Toluol-Sulfonat (58 mg, 0,232 mmol) und N-Methylinorpholin (23 mg, 0,23 mmol). Die Suspension wurde unter Argon-Atmosphäre gerührt, bis die klare Lösung A erhalten wurde. D-Ornithin wurde nach Standardverfahren N-terminal mit Fmoc und C-terminal mit einer Allylgruppe geschützt (= Fmoc-D-Orn-OAll). Die klare Lösung A wurde zu einer Lösung von Fmoc-D-Orn-OALL (100 mg, 0,23 mmol) und N-Methylmorpholin (23 mg, 0,23 mmol) in DMF (2,3 ml) gegeben. Das Reaktionsgemisch wurde 12h unter Argon-Atmosphäre gerührt. Das Lösungsmittel wurde im Vakuum entfernt, den Rückstand nahm man in Methanol (2ml) auf. Man rührte 1h, filtrierte den Feststoff ab und wusch mit Methanol nach (5 ml). Das Rohprodukt wurde durch Säulenchromatographie gereinigt (Chloroform : Methanol 97:3, 1% Ameinsensäure). Man erhielt 103mg Fmoc-D-Orn(TO)-OAll in Form eines orange-farbenen Feststoffes, entsprechend einer Ausbeute von 55%.
Rf (Chloroform : Methanol 80:20, 1% Ameinsensäure) = 0,56

### Beispiel 4: Herstellung von Nδ-[9-(Fluorenylmethoxycarbonyl)-D-ornithin]-Nα-[2-(1-carboxynethyl-1H-quinolin-4-ylidenmethyl)-3-methyl-benzothiazol-3-ium bromid] OH (Fmoc-D-Orn(TO)-OH) 2

Zu einer Lösung von Fmoc-D-Orn(TO)-OAll (81 mg, 0,1 mmol) in THF (5 ml) gab man N-Methylanilin (11,3 mg, 0,1 mmol) und entgaste die Lösung mehrmals. Nach Zugabe von [Pd(PPh₃)₄] (12 mg, 0,01 mmol) rührte man die Mischung 14 h unter Lichtausschluß. Das Lösungsmittel wurde im Vakuum entfernt. Den Rückstand löste man in Methanol (4 ml) und filtrierte das Präzipitat ab. Das Rohprodukt wurde säulenchromatographisch gereinigt (Chloroform : Methanol 97:3, 1% Ameisensäure). Man erhielt 52 mg des Produktes Fmoc-D-Om(TO)-OH (1) in Form eines orange-farbenen Feststoffes, entsprechend einer Ausbeute von 65%.
Rf (Chloroform : Methanol 80:20, 1% Ameisensäure) = 0,22

### Beispiel 5: Festphasensynthese von Sonden

### Beladung der NovaSyn® TGR Festphase:

Die Festphase (500 mg, 0,29 mmol/g) wurde gewaschen (3x Dichlormethan, 3x Dimethylformamid, 3x Dichlormethan, 3x Dimethylformamid). Man ließ die Festphase 30 min in Dimethylformamid (10 ml) quellen. Zur Präaktivierung gab man (Benzotriazol-1-yloxy)tripyrrolidinophosphoniumhexafluorophosphat (PyBOP^{®} 301,2 mg, 0,58 mmol) und N-Methylmorpholin (87,7 mg, 0,87 mmol) zu einer Lösung von Fmoc-geschütztem Glycin (172,3 mg, 0,58 mmol) in Dimethylformamid (5,8 ml). Nach 3 min Inkubation wurde die erhaltene Lösung mit der Festphase vermengt. Nach weiteren 4 h Inkubation wusch man die Festphase (3x Dimethylformamid, 3x Dichlormethan, 3x Dimethylformamid) und blockierte Gruppen, die nicht reagiert hatten, mit einer Lösung von Acetanhydrid in Pyridin (1:4, 5 ml). Nach 5 min wiederholte man den Blockierungsschritt. Die Festphase wurde gewaschen (3x Dimethylformamid, 3x Dichlormethan, 3x Dimethylformamid und 5x Dichlormethan) und schließlich im Vakuum getrocknet.

### Synthese unter Verwendung eines automatisierten Synthesizers

Die Synthese der Sonden am automatisierten Synthesizer (Intavis ResPep der Firma Intavis AG) wurde gemäß folgender allgemeiner Arbeitsvorschrift durchgeführt:
Man ließ die beladene Festphase in Dimethylformamid quellen (2 ml). Nach 30 min wurde die gequollene Festphase zum Synthesizer transferiert. Man wusch 2x mit Dimethylformamid (180 µl).
Fmoc-Abspaltung: Man versetzte die Festphase mit Dimethylformamid/Piperidin (4:1, 100 µl). Nach 2 min wurde die Prozedur wiederholt, abschließend wusch man die Festphase mit DMF (1x 180 µl, 3x 100 µl und 1x 180 µl).
Kopplung: Die Festphase wurde in einer Lösung von 4 Äquivalenten des zu koppelnden, Fmoc-geschützten Bausteins und 8 Äquivalenten N-Methyl-morpholin in Dimethylformamid (0,3M Konzentration des Bausteins in NMP, der 8 min durch Inkubation mit 3,6 Äquivalenten 1H-Benzotriazolium-1-[bis(dimethylamino)methylen] -5chloro-hexafluorophosphat (HCTU) präaktiviert wurde) suspendiert. Nach 30 min wusch man die Festphase (2x 180 µl Dimethylformamid).
Capping: Das Capping erfolgte durch 3 min Behandlung mit Acetanhydrid/2,6-Lutidin/Pyridin (5:6:89, 100µl). Anschließend wurde die Festphase gewaschen (2x 180 µl Dimethylformamid, 1x 100µl Dimethylformamid).

Bei der Kopplung der Verbindungen 1 und 2 verfuhr man gemäß der oben angegebenen Vorschrift, wobei PPTS (2 mg, 8 µmol) hinzugefügt wurde um die Löslichkeit zu verbessern. Die Kopplung wurde zweimal durchgeführt.
Abspaltung von der Festphase: Eine Lösung von Cystinmethylesterhydrochlorid (5 mg, 29 µmol) in Trifluoressigsäure/m-Kresol/Wasser (37:2:1, 1 ml) wurde 40 min lang durch die vorher getrocknete Festphase geleitet. Man wusch die Festphase mit Trifluoressigsäure (500 µl). Die vereinigten Filtrate wurden im Vakuum konzentriert.
Reinigung: Zu der konzentrierten Abspaltungslösung gab man kalten Diethylether. Das Präzipitat wurde zentrifugiert und der Überstand verworfen. Den Rückstand nahm man in Wasser auf und führte eine Vorreinigung mit einer SepPak^{®} C18-Säule durch, die mit Wasser äquilibriert wurde. Die nach Gradientenelution erhaltenen farbigen Eluate (1x 20:80 Acetonitril:H₂O: 0,1% Trifluoressigsäure; 1x 40:60 Acetonitril:H₂O: 1% Trifluoressigsäure; 1x 80:20 Acetonitril:H₂O:0,1% Trifluoressigsäure; 1x 80:20 Acetonitril:H₂O: 0,1% Trifluoressigsäure; jeweils 2 ml) wurden mittels HPLC und MALDI-TOF Massenspektrometrie analysiert und durch semipräparative HPLC gereinigt.

### Beispiele für die Verwendung der Sonden

Die folgenden zwei Beispiele demonstrieren exemplarisch, wie die Sonden für die homogene DNA-Detektion eingesetzt werden können und zeigen einen Vergleich mit dem Stand der Technik.

### Beispiel 6: Homogener DNA-Nachweis mit Thiazolorange-L-Ornithin- und Thiazolorange-D-Ornithin-Sonden im Vergleich zu Thiazolorange-Aminoethylglycin-Sonden

Die Fluoreszenzspektren wurden bei 25 °C mit einem Spektrometer LS 50B der Firma Perkin-Elmer aufgenommen. Die Messungen erfolgten in Phosphatpuffer, pH 7 (100 mM NaCl, 10 mM Na₂HPO₄, 0,1 mM EDTA). Für die Hybridisierung verwendete man je 1 nmol des DNA-Targets und der jeweiligen PNA-Sonde in 1 ml Pufferlösung. Die verwendete PNA-Sonde besaß die allgemeine Sequenz Ac-gccgta-O-tagccg-Gly-NH2, wobei O für den Farbstoff Thiazolorange steht, der entweder an ein Aminoethylglycin-, D-Ornitin- oder L-Ornithin-Rückgrat gebunden war. Das DNA-Target besaß die allgemeine Sequenz 5'-CGGCTAXTACGGC, wobei X die im Duplex dem Basensurrogat gegenüber liegende Nukleobase, also entweder C, T, A oder G war. Abbildung 1 zeigt das Verhältnis zwischen den gemessenen Fluoreszenzintensitäten der Sonden vor und nach Zugabe der DNA-Targets in Abhängigkeit von der Rückgratstruktur, an die das Thiazolorange gebunden war sowie in Abhängigkeit von der dem Basensurrogat gegenüber liegenden Base. Die Ergebnisse zeigen beispielhaft, dass das L-Ornithin-Rückgrat in allen Fällen ein höheres Fluoreszenzsignal ergibt als das Aminoethylglycin-Rückgrat. Das D-Ornithin-Rückgrat gibt in allen vier Fällen schwächere Fluoreszenzsignale als das L-Ornithin-Rückgrat. Im Vergleich zum Aminoethylglycin-Rückgrat gibt das D-Ornithin-Rückgat in drei Fällen eine höhere Fluoreszenzintensität als das Aminoethylglycin-Rückgrat, in einem Fall sind die Intensitäten gleich. Das Beispiel zeigt auch, dass durch geeignete Auswahl der Sondensequenz der Fluoreszenzanstieg bei der Hybridisierung maximiert werden kann.

### Beispiel 7: Nachweis von Einzelbasenmutationen mit Thiazolorange-L-Ornithin- und Thiazolorange-D-Ornithin-Sonden im Vergleich zu Thiazolorange-Aminoethylglycin-Sonden

Die Fluoreszenzspektren wurden bei 25 °C mit einem Spektrometer LS 50B der Firma Perkin-Elmer aufgenommen. Die Messungen erfolgten in Phosphatpuffer, pH 7 (100 mM NaCl, 10 mM Na₂HPO₄, 0,1 mM EDTA). Für die Hybridisierung verwendete man je 1 nmol des DNA-Targets und der jeweiligen PNA-Sonde in 1 ml Pufferlösung. Die verwendete PNA-Sonde besaß die allgemeine Sequenz Ac-gccgta-O-tagccg-Gly-NH2, wobei O für den Farbstoff Thiazolorange steht, der entweder an ein Aminoethylglycin-, D-Ornithin- oder L-Ornithin-Rückgrat gebunden war. Das DNA-Target besaß die allgemeine Sequenz 5'-CGGCTXTTACGGC, wobei X die Einzelbasenmutation im DNA-Target, also entweder C, T, A oder G war. Im Fall von X=A handelt es sich um den perfekt gepaarten Duplex. Abbildung 2 zeigt das Verhältnis zwischen den gemessenen Fluoreszenzintensitäten der Sonden nach und vor Zugabe der DNA-Targets in Abhängigkeit von der Rückgratstruktur, an die das Thiazolorange gebunden war sowie in Abhängigkeit von der im Target vorkommenden Einzelbasenmutation. Die Ergebnisse zeigen, dass Ornithin-Sonden generell eine höhere Spezifität in der Erkennung von Einzelbasenmutationen besitzen als Aminoethylglycin-Sonden. Die D-Ornithin-Sonden zeigen generell schwächere Fluoreszenzsignale als die L-Ornithin-Sonden. Während das Signalverhältnis zwischen dem perfekt gepaarten Duplex und den verschiedenen, einen Mismatch enthaltenen Duplices im Fall von L-Omitin 6-12 beträgt, wird bei D-Ornithin ein Verhältnis von 8-12 beobachtet. Das Beispiel demonstriert die generell höhere Spezifität der D-Ornithin-Sonden.

### Beispiel 8: Bestimmung von Schmelztemperaturen verschiedener Duplexe mit Thiazolorange-Aminoethylglycin-, Thiazolorange-L-Ornithin- und Thiazolorange-D-Ornithin-Sonden

Die Schmelzkurven der Sonden-DNA-Komplexe wurden mit einem UV-VIS-Spektrometer der Firma Varian (Cary 100) in Wasser aufgenommen. Die Messung erfolgte mit einem Gradienten von 0,3°C pro Minute, wobei je zweimal von 85°C - 15°C und je zweimal von 15°C-85°C gemessen wurde. Der Wendepunkt der Schmelzkurve wurde als Schmelztemperatur des jeweiligen Duplexes bestimmt. Man verwendete zwei DNA-Targets mit der Sequenz 5'-AGTGAAATGTTATACGAAACT-3' (match-Target) und 5'-GAAATGCTATACGAA-3' (mismatch-Target). Als Sonde verwendete man Verbindungen der allgemeinen Sequenz N-ttcgtat-O-acatttc-Lys-C, wobei O für den Farbstoff Thiazolorange steht, der entweder an ein Aminoethylglycin-, D-Ornitin- oder L-Ornithin-Rückgrat gebunden war. Folgende Schmelztemperaturen wurden für die verschiedenen Duplexe ermittelt:

| | Aminoethylglycin | L-Ornithin | D-Ornithin |
|---|---|---|---|
| Tm (match) | 59°C | 59°C | 55°C |
| Tm (mismatch) | 37°C | 38°C | 35°C |
| ΔTm | 22°C | 20°C | 21°C |

Das Beispiel zeigt, dass durch die veränderten (aufgeweiteten) Rückgratstrukturen im Falle der Ornithin-Sonden keine signifikante Destabilisierung der Duplexe erfolgt. Zudem sind die Differenzen der Schmelztemperaturen von match- und mismatch-Duplexen im Rahmen der experimentellen Fehlergrenzen gleich. Dies Beispiel zeigt, dass die durch das Ornithin-Rückgrat verbesserte Unterscheidung zwischen match- und mismatch-Duplexen nicht auf veränderte Schmelzpunkte, sondern auf die Veränderung der Umgebung des Basensurrogates zurückzuführen ist. Hierdurch wird die Anwendbarkeit der Sonden zur Einzelbasenunterscheidung in einem weiten Temperaturbereich ermöglicht.

## Patentansprüche

1. PNA-Sonde der allgemeinen Formel: 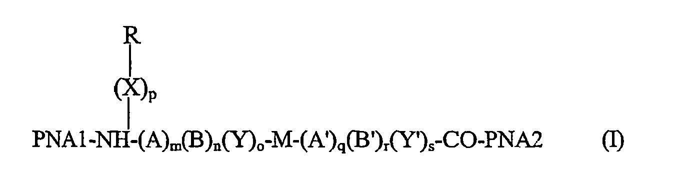 in der
PNA1 ein Peptidnukleinsäurestrang beliebiger Sequenz und Länge, bevorzugt eine Sequenz von 2-100 Basen, besonders bevorzugt zwischen 2-10 Basen ist,
PNA2 ein Peptidnukleinsäurestrang beliebiger Sequenz und Länge, bevorzugt eine Sequenz von 2-100 Basen, besonders bevorzugt zwischen 2-10 Basen ist,
A eine Methylengruppe oder eine substituierte Kohlenstoffeinheit des Typs CHR, oder CR2 (wobei R Wasserstoff oder ein organischer Rest ist, bevorzugt eine substituierte oder unsubstituierte Methylgruppe, weiterhin bevorzugt eine substituierte oder unsubstituierte Alkylgruppe, weiterhin bevorzugt eine substituierte oder unsubstituierte Phenylgruppe), bevorzugt eine Methylengruppe ist,
B eine Methylengruppe oder eine substituierte Kohlenstoffeinheit des Typs CHR, oder CR2 (wobei R Wasserstoff oder ein organischer Rest ist, bevorzugt eine substituierte oder unsubstituierte Methylgruppe, weiterhin bevorzugt eine substituierte oder unsubstituierte Alkylgruppe, weiterhin bevorzugt eine substituierte oder unsubstituierte Phenylgruppe), eine substituierte oder unsubstituierte Aminogruppe oder ein Atom der 5. Oder 6. Hauptgruppe des Periodensystems, bevorzugt Sauerstoff oder Schwefel, besonders bevorzugt eine Methylengruppe ist,
C eine Methylengruppe oder eine substituierte Kohlenstoffeinheit des Typs CHR, oder CR2 (wobei R Wasserstoff oder ein organischer Rest ist, bevorzugt eine substituierte oder unsubstituierte Methylgruppe, weiterhin bevorzugt eine substituierte oder unsubstituierte Alkylgruppe, weiterhin bevorzugt eine substituierte oder unsubstituierte Phenylgruppe), eine substituierte oder unsubstituierte Aminogruppe oder ein Atom der 5. Oder 6. Hauptgruppe des Periodensystems, bevorzugt Sauerstoff oder Schwefel, besonders bevorzugt eine Methylengruppe ist,
m eine natürliche Zahl von 1-5, bevorzugt 1 ist,
n eine natürliche Zahl von 1-5, bevorzugt 1 ist,
o eine natürliche Zahl von 1-5, bevorzugt 1 ist,
X eine Methylengruppe oder eine substituierte Kohlenstoffeinheit des Typs CHR, oder CR2 (wobei R Wasserstoff oder ein organischer Rest ist, bevorzugt eine substituierte oder unsubstituierte Methylgruppe, weiterhin bevorzugt eine substituierte oder unsubstituierte Alkylgruppe, weiterhin bevorzugt eine substituierte oder unsubstituierte Phenylgruppe), eine substituierte oder unsubstituierte Aminogruppe des Typs NH oder NR (wobei R Wasserstoff oder ein organischer Rest ist, bevorzugt eine substituierte oder unsubstituierte Methylgruppe, weiterhin bevorzugt eine substituierte oder unsubstituierte Alkylgruppe, weiterhin bevorzugt eine substituierte oder unsubstituierte Phenylgruppe) oder ein Atom der 5. oder 6. Hauptgruppe des Periodensystems, bevorzugt Sauerstoff oder Schwefel, besonders bevorzugt eine NH-Gruppe ist,
p eine natürliche Zahl von 0 bis 10, bevorzugt eine Zahl von 1-6 ist,
R ein fluoreszentes, universelles Basensurrogat, **dadurch gekennzeichnet, dass** es fluoreszent und zur Interkalation in DNA befähigt ist, bevorzugt ein Cyaninfarbstoff, weiterhin bevorzugt Ethidium, weiterhin bevorzugt ein substituiertes Anthracen, weiterhin bevorzugt ein Acridin-Farbstoff, weiterhin bevorzugt ein Farbstoff ausgewählt aus den von der Firma Molecular Probes vertriebenen Farbstoffe PO-PRO-1, BO-PRO-1, YO-PRO-1, TO-PRO-1, JO-PRO-1, PO-PRO-1, PO-PRO-3, LO-PRO-1, BO-PRO-3, YO-PRO-3, TO-PRO-3, TO-PRO-5 (sowie von diesen Verbindungen abgeleitete Fluorophore mit veränderter Linkerstruktur), besonders bevorzugt Thiazolorange ist,
M eine substituierte Kohlenstoffeinheit des Typs CH, oder CR (wobei R Wasserstoff oder ein organischer Rest ist, bevorzugt eine substituierte oder unsubstituierte Methylgruppe, weiterhin bevorzugt eine substituierte oder unsubstituierte Alkylgruppe, weiterhin bevorzugt eine substituierte oder unsubstituierte Phenylgruppe), eine substituierte Aminogruppe des Typs NH oder NR (wobei R Wasserstoff oder ein organischer Rest ist, bevorzugt eine substituierte oder unsubstituierte Methylgruppe, weiterhin bevorzugt eine substituierte oder unsubstituierte Alkylgruppe, weiterhin bevorzugt eine substituierte oder unsubstituierte Phenylgruppe) oder ein Atom der 5. Oder 6. Hauptgruppe des Periodensystems, bevorzugt Sauerstoff oder Schwefel, besonders bevorzugt eine CH-Gruppe ist,
A' eine Methylengruppe oder eine substituierte Kohlenstoffeinheit des Typs CHR, oder CR2 (wobei R Wasserstoff oder ein organischer Rest ist, bevorzugt eine substituierte oder unsubstituierte Methylgruppe, weiterhin bevorzugt eine substituierte oder unsubstituierte Alkylgruppe, weiterhin bevorzugt eine substituierte oder unsubstituierte Phenylgruppe), eine substituierte oder unsubstituierte Aminogruppe oder ein Atom der 5. oder 6. Hauptgruppe des Periodensystems, bevorzugt Sauerstoff oder Schwefel, besonders bevorzugt eine Methylengruppe ist,
B' eine Methylengruppe oder eine substituierte Kohlenstoffeinheit des Typs CHR, oder CR2 (wobei R Wasserstoff oder ein organischer Rest ist, bevorzugt eine substituierte oder unsubstituierte Methylgruppe, weiterhin bevorzugt eine substituierte oder unsubstituierte Alkylgruppe, weiterhin bevorzugt eine substituierte oder unsubstituierte Phenylgruppe), eine substituierte oder unsubstituierte Aminogruppe oder ein Atom der 5. oder 6. Hauptgruppe des Periodensystems, bevorzugt Sauerstoff oder Schwefel, besonders bevorzugt eine Methylengruppe ist,
Y eine Methylengruppe oder eine substituierte Kohlenstoffeinheit des Typs CHR, oder CR2 (wobei R Wasserstoff oder ein organischer Rest ist, bevorzugt eine substituierte oder unsubstituierte Methylgruppe, weiterhin bevorzugt eine substituierte oder unsubstituierte Alkylgruppe, weiterhin bevorzugt eine substituierte oder unsubstituierte Phenylgruppe), besonders bevorzugt eine Methylengruppe ist,
q eine natürliche Zahl von 0-5, bevorzugt 0 ist,
r eine natürliche Zahl von 0-5, bevorzugt 0 ist,
s eine natürliche Zahl von 0-5, bevorzugt 0 ist.

2. Sonde der allgemeinen Formel 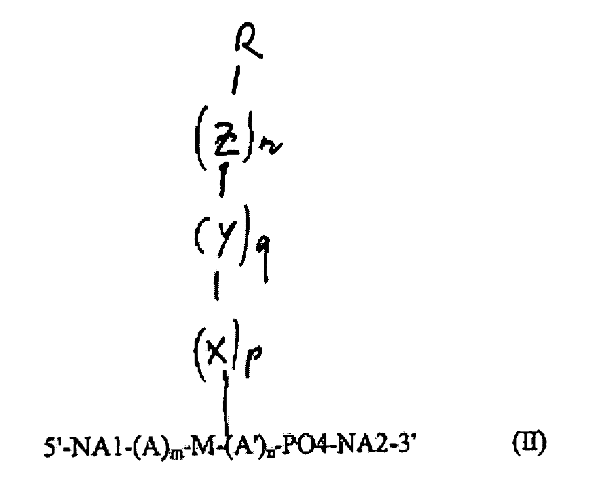 in der
5'-NA1 eine Nukleinsäure oder ein Nukleinsäureanalogon beliebiger Sequenz und Länge, die mit einer 3'-terminalen Phosphatgruppe endet, bevorzugt eine Nukleinsäure-Sequenz von 3-100 Basen Länge, besonders bevorzugt eine Nukleinsäure-Sequenz von 3-20 Basen Länge, ganz besonders bevorzugt eine Nukleinsäure-Sequenz von 3-15 Basen Länge ist,
NA2-3' eine Nukleinsäure oder ein Nukleinsäureanalogon beliebiger Sequenz und Länge, bevorzugt eine Nukleinsäure-Sequenz von 3-100 Basen Länge, besonders bevorzugt eine Nukleinsäure-Sequenz von 3-20 Basen Länge, ganz besonders bevorzugt eine Nukleinsäure-Sequenz von 3-15 Basen Länge ist,
A eine Methylengruppe oder eine substituierte Kohlenstoffeinheit des Typs CHR, oder CR2 (wobei R Wasserstoff oder ein organischer Rest ist, bevorzugt eine substituierte oder unsubstituierte Methylgruppe, weiterhin bevorzugt eine substituierte oder unsubstituierte Alkylgruppe, weiterhin bevorzugt eine substituierte oder unsubstituierte Phenylgruppe), bevorzugt eine Methylengruppe ist,
m eine natürliche Zahl von 0-5 ist,
X eine Methylengruppe oder eine substituierte Kohlenstoffeinheit des Typs CHR, oder CR2 (wobei R Wasserstoff oder ein organischer Rest ist, bevorzugt eine substituierte oder unsubstituierte Methylgruppe, weiterhin bevorzugt eine substituierte oder unsubstituierte Alkylgruppe, weiterhin bevorzugt eine substituierte oder unsubstituierte Phenylgruppe), eine substituierte oder unsubstituierte Aminogruppe des Typs NH oder NR (wobei R Wasserstoff oder ein organischer Rest ist, bevorzugt eine substituierte oder unsubstituierte Methylgruppe, weiterhin bevorzugt eine substituierte oder unsubstituierte Alkylgruppe, weiterhin bevorzugt eine substituierte oder unsubstituierte Phenylgruppe) oder ein Atom der 5. Oder 6. Hauptgruppe des Periodensystems, bevorzugt Sauerstoff oder Schwefel, besonders bevorzugt eine Methylengruppe ist,
Y eine Methylengruppe oder eine substituierte Kohlenstoffeinheit des Typs CHR, oder CR2 (wobei R Wasserstoff oder ein organischer Rest ist, bevorzugt eine substituierte oder unsubstituierte Methylgruppe, weiterhin bevorzugt eine substituierte oder unsubstituierte Alkylgruppe, weiterhin bevorzugt eine substituierte oder unsubstituierte Phenylgruppe), eine substituierte oder unsubstituierte Aminogruppe des Typs NH oder NR (wobei R Wasserstoff oder ein organischer Rest ist, bevorzugt eine substituierte oder unsubstituierte Methylgruppe, weiterhin bevorzugt eine substituierte oder unsubstituierte Alkylgruppe, weiterhin bevorzugt eine substituierte oder unsubstituierte Phenylgruppe) oder ein Atom der 5. Oder 6. Hauptgruppe des Periodensystems, bevorzugt Sauerstoff oder Schwefel, besonders bevorzugt eine NH-Gruppe ist,
Z eine Methylengruppe oder eine substituierte Kohlenstoffeinheit des Typs CHR, oder CR2 (wobei R Wasserstoff oder ein organischer Rest ist, bevorzugt eine substituierte oder unsubstituierte Methylgruppe, weiterhin bevorzugt eine substituierte oder unsubstituierte Alkylgruppe, weiterhin bevorzugt eine substituierte oder unsubstituierte Phenylgruppe), eine substituierte oder unsubstituierte Aminogruppe des Typs NH oder NR (wobei R Wasserstoff oder ein organischer Rest ist, bevorzugt eine substituierte oder unsubstituierte Methylgruppe, weiterhin bevorzugt eine substituierte oder unsubstituierte Alkylgruppe, weiterhin bevorzugt eine substituierte oder unsubstituierte Phenylgruppe) oder ein Atom der 5. oder 6. Hauptgruppe des Periodensystems, bevorzugt Sauerstoff oder Schwefel, besonders bevorzugt eine Methylengruppe ist,
p eine natürliche Zahl von 0 bis 10, bevorzugt eine Zahl von 1-6, besonders bevorzugt 1 ist,
q eine natürliche Zahl von 0 bis 10, bevorzugt eine Zahl von 1-6, besonders bevorzugt 1 ist,
r eine natürliche Zahl von 0 bis 10, bevorzugt eine Zahl von 1-6 ist,
R ein fluoreszentes, universelles Basensurrogat, **dadurch gekennzeichnet, dass** es fluoreszent und zur Interkalation in DNA befähigt ist, bevorzugt ein Cyaninfarbstoff, weiterhin bevorzugt Ethidium, weiterhin bevorzugt ein substituiertes Anthracen, weiterhin bevorzugt ein Acridin-Farbstoff, weiterhin bevorzugt ein Farbstoff ausgewählt aus den von der Firma Molecular Probes vertriebenen Farbstoffe PO-PRO-1, BO-PRO-1, YO-PRO-1, TO-PRO-1, JO-PRO-1, PO-PRO-1, PO-PRO-3, LO-PRO-1, BO-PRO-3, YO-PRO-3, TO-PRO-3, TO-PRO-5 (sowie von diesen Verbindungen abgeleitete Fluorophore mit veränderter Linkerstruktur), besonders bevorzugt Thiazolorange ist,
M eine substituierte Kohlenstoffeinheit des Typs CH, oder CR (wobei R Wasserstoff oder ein organischer Rest ist, bevorzugt eine substituierte oder unsubstituierte Methylgruppe, weiterhin bevorzugt eine substituierte oder unsubstituierte Alkylgruppe, weiterhin bevorzugt eine substituierte oder unsubstituierte Phenylgruppe), eine substituierte Aminogruppe des Typs NH oder NR (wobei R Wasserstoff oder ein organischer Rest ist, bevorzugt eine substituierte oder unsubstituierte Methylgruppe, weiterhin bevorzugt eine substituierte oder unsubstituierte Alkylgruppe, weiterhin bevorzugt eine substituierte oder unsubstituierte Phenylgruppe) oder ein Atom der 5. oder 6. Hauptgruppe des Periodensystems, bevorzugt Sauerstoff oder Schwefel, besonders bevorzugt eine CH-Gruppe ist,
A' eine Methylengruppe oder eine substituierte Kohlenstoffeinheit des Typs CHR, oder CR2 (wobei R Wasserstoff oder ein organischer Rest ist, bevorzugt eine substituierte oder unsubstituierte Methylgruppe, weiterhin bevorzugt eine substituierte oder unsubstituierte Alkylgruppe, weiterhin bevorzugt eine substituierte oder unsubstituierte Phenylgruppe), eine substituierte oder unsubstituierte Aminogruppe oder ein Atom der 5. oder 6. Hauptgruppe des Periodensystems, bevorzugt Schwefel, bevorzugt eine Methylengruppe ist,
n eine natürliche Zahl von 0-5 ist.

3. Sonde gemäß einem der Ansprüche 1 und/oder 2, **dadurch gekennzeichnet, dass** mehrere Einheiten der allgemeinen Struktur (I) oder (II) kovalent verknüpft werden und eine Sonde mit multiplen Markierungen erhalten wird.

4. Verfahren zur Herstellung der Sonde gemäß einem der Ansprüche 1-3 durch Festphasensynthese, bevorzugt durch automatisierte Festphasensynthese.

5. Verwendung der Sonde gemäß einem der Ansprüche 1-3 zur Durchführung von Nukleinsäure-Assays, bevorzugt zur Durchführung homogener Nukleinsäure-Assays.

6. Verwendung der Sonde gemäß einem der Ansprüche 1-3 zur Durchführung einer PCR-Reaktion, bevorzugt zur Durchführung einer quantitativen PCR-Reaktion.

7. Verwendung der Sonde gemäß einem der Ansprüche 1-3 zur Durchführung von Multiplex Assays, **dadurch gekennzeichnet, dass** verschiedene Sonden durch verschiedene fluoreszente Basensurrogate markiert werden.

8. Sonde gemäß Anspruch 1 oder 3, wobei die Sonde L-Omitin enthält, an das ein fluoreszentes Basensurrogat, bevorzugt Thiazolorange, gebunden ist.

9. Sonde gemäß Anspruch 1 oder 3, wobei die Sonde D-Ornitin enthält, an das ein fluoreszentes Basensurrogat, bevorzugt Thiazolorange, gebunden ist.

10. Testkit, das diejenigen Reagenzien enthält, die zur Durchführung der Assays nach einem der Ansprüche 5-7 benötigt werden.

## Claims

1. A PNA probe of the general formula: 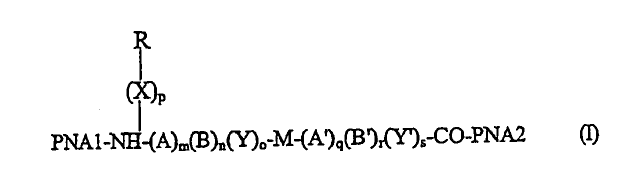 in which
PNA1 is a peptide nucleic acid strand of any sequence and length, preferably a sequence of 2-100 bases, particularly preferably between 2-10 bases,
PNA2 is a peptide nucleic acid strand of any sequence and length, preferably a sequence of 2-100 bases, particularly preferably between 2-10 bases,
A is a methylene group or a substituted carbon moiety of the CHR or CR2 type (wherein R is hydrogen or an organic radical, preferably a substituted or unsubstituted methyl group, furthermore preferably a substituted or unsubstituted alkyl group, furthermore preferably a substituted or unsubstituted phenyl group), preferably a methylene group,
B is a methylene group or a substituted carbon moiety of the CHR or CR2 type (wherein R is hydrogen or an organic radical, preferably a substituted or unsubstituted methyl group, furthermore preferably a substituted or unsubstituted alkyl group, furthermore preferably a substituted or unsubstituted phenyl group), a substituted or unsubstituted amino group or an atom of the 5th or 6th main group of the Periodic Table, preferably oxygen or sulfur, particularly preferably a methylene group,
C is a methylene group or a substituted carbon moiety of the CHR or CR2 type (wherein R is hydrogen or an organic radical, preferably a substituted or unsubstituted methyl group, furthermore preferably a substituted or unsubstituted alkyl group, furthermore preferably a substituted or unsubstituted phenyl group), a substituted or unsubstituted amino group or an atom of the 5th or 6th main group of the Periodic Table, preferably oxygen or sulfur, particularly preferably a methylene group,
m is a natural number from 1-5, preferably 1,
n is a natural number from 1-5, preferably 1,
o is a natural number from 1-5, preferably 1,
X is a methylene group or a substituted carbon moiety of the CHR or CR2 type (wherein R is hydrogen or an organic radical, preferably a substituted or unsubstituted methyl group, furthermore preferably a substituted or unsubstituted alkyl group, furthermore preferably a substituted or unsubstituted phenyl group), a substituted or unsubstituted amino group of the NH or NR type (wherein R is hydrogen or an organic radical, preferably a substituted or unsubstituted methyl group, furthermore preferably a substituted or unsubstituted alkyl group, furthermore preferably a substituted or unsubstituted phenyl group) or an atom of the 5th or 6th main group of the Periodic Table, preferably oxygen or sulfur, particularly preferably an NH group,
p is a natural number from 0 to 10, preferably a number from 1-6,
R is a fluorescent, universal base surrogate, **characterized in that** it is fluorescent and able to intercalate into DNA, preferably a cyanine dye, furthermore preferably ethidium, furthermore preferably a substituted anthracene, furthermore preferably an acridine dye, furthermore preferably a dye selected from the dyes sold by Molecular Probes, PO-PRO-1, BO-PRO-1, YO-PRO-1, TO-PRO-1, JO-PRO-1, PO-PRO-1, PO-PRO-3, LO-PRO-1, BO-PRO-3, YO-PRO-3, TO-PRO-3, TO-PRO-5 (and fluorophores derived from these compounds, which have a modified linker structure), particularly preferably thiazole orange,
M is a substituted carbon moiety of the CH or CR type (wherein R is hydrogen or an organic radical, preferably a substituted or unsubstituted methyl group, furthermore preferably a substituted or unsubstituted alkyl group, furthermore preferably a substituted or unsubstituted phenyl group), a substituted amino group of the NH or NR type (wherein R is hydrogen or an organic radical, preferably a substituted or unsubstituted methyl group, furthermore preferably a substituted or unsubstituted alkyl group, furthermore preferably a substituted or unsubstituted phenyl group) or an atom of the 5th or 6th main group of the Periodic Table, preferably oxygen or sulfur, particularly preferably a CH group,
A' is a methylene group or a substituted carbon moiety of the CHR or CR2 type (wherein R is hydrogen or an organic radical, preferably a substituted or unsubstituted methyl group, furthermore preferably a substituted or unsubstituted alkyl group, furthermore preferably a substituted or unsubstituted phenyl group), a substituted or unsubstituted amino group or an atom of the 5th or 6th main group of the Periodic Table, preferably oxygen or sulfur, particularly preferably a methylene group,
B' is a methylene group or a substituted carbon moiety of the CHR or CR2 type (wherein R is hydrogen or an organic radical, preferably a substituted or unsubstituted methyl group, furthermore preferably a substituted or unsubstituted alkyl group, furthermore preferably a substituted or unsubstituted phenyl group), a substituted or unsubstituted amino group or an atom of the 5th or 6th main group of the Periodic Table, preferably oxygen or sulfur, particularly preferably a methylene group,
Y' is a methylene group or a substituted carbon moiety of the CHR or CR2 type (wherein R is hydrogen or an organic radical, preferably a substituted or unsubstituted methyl group, furthermore preferably a substituted or unsubstituted alkyl group, furthermore preferably a substituted or unsubstituted phenyl group), particularly preferably a methylene group,
q is a natural number from 0-5, preferably 0,
r is a natural number from 0-5, preferably 0,
s is a natural number from 0-5, preferably 0.

2. A probe of the general formula 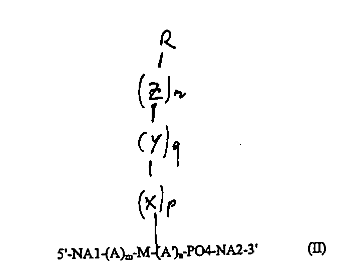 in which
5'-NA1 is a nucleic acid or a nucleic acid analog of any sequence and length, which ends in a 3'-terminal phosphate group, preferably a nucleic acid sequence of 3-100 bases in length, particularly preferably a nucleic acid sequence of 3-20 bases in length, very particularly preferably a nucleic acid sequence of 3-15 bases in length,
NA2-3' is a nucleic acid or a nucleic acid analog of any sequence and length, preferably a nucleic acid sequence of 3-100 bases in length, particularly preferably a nucleic acid sequence of 3-20 bases in length, very particularly preferably a nucleic acid sequence of 3-15 bases in length,
A is a methylene group or a substituted carbon moiety of the CHR or CR2 type (wherein R is hydrogen or an organic radical, preferably a substituted or unsubstituted methyl group, furthermore preferably a substituted or unsubstituted alkyl group, furthermore preferably a substituted or unsubstituted phenyl group), preferably a methylene group,
m is a natural number from 0-5,
X is a methylene group or a substituted carbon moiety of the CHR or CR2 type (wherein R is hydrogen or an organic radical, preferably a substituted or unsubstituted methyl group, furthermore preferably a substituted or unsubstituted alkyl group, furthermore preferably a substituted or unsubstituted phenyl group), a substituted or unsubstituted amino group of the NH or NR type (wherein R is hydrogen or an organic radical, preferably a substituted or unsubstituted methyl group, furthermore preferably a substituted or unsubstituted alkyl group, furthermore preferably a substituted or unsubstituted phenyl group) or an atom of the 5th or 6th main group of the Periodic Table, preferably oxygen or sulfur, particularly preferably a methylene group,
Y is a methylene group or a substituted carbon moiety of the CHR or CR2 type (wherein R is hydrogen or an organic radical, preferably a substituted or unsubstituted methyl group, furthermore preferably a substituted or unsubstituted alkyl group, furthermore preferably a substituted or unsubstituted phenyl group), a substituted or unsubstituted amino group of the NH or NR type (wherein R is hydrogen or an organic radical, preferably a substituted or unsubstituted methyl group, furthermore preferably a substituted or unsubstituted alkyl group, furthermore preferably a substituted or unsubstituted phenyl group) or an atom of the 5th or 6th main group of the Periodic Table, preferably oxygen or sulfur, particularly preferably an NH group,
Z is a methylene group or a substituted carbon moiety of the CHR or CR2 type (wherein R is hydrogen or an organic radical, preferably a substituted or unsubstituted methyl group, furthermore preferably a substituted or unsubstituted alkyl group, furthermore preferably a substituted or unsubstituted phenyl group), a substituted or unsubstituted amino group of the NH or NR type (wherein R is hydrogen or an organic radical, preferably a substituted or unsubstituted methyl group, furthermore preferably a substituted or unsubstituted alkyl group, furthermore preferably a substituted or unsubstituted phenyl group) or an atom of the 5th or 6th main group of the Periodic Table, preferably oxygen or sulfur, particularly preferably a methylene group,
p is a natural number of from 0 to 10, preferably a number from 1-6, particularly preferably 1,
q is a natural number of from 0 to 10, preferably a number from 1-6, particularly preferably 1,
r is a natural number from 0 to 10, preferably a number from 1-6,
R is a fluorescent, universal base surrogate, **characterized in that** it is fluorescent and able to intercalate into DNA, preferably a cyanine dye, furthermore preferably ethidium, furthermore preferably a substituted anthracene, furthermore preferably an acridine dye, furthermore preferably a dye selected from the dyes sold by Molecular Probes, PO-PRO-1, BO-PRO-1, YO-PRO-1, TO-PRO-1, JO-PRO-1, PO-PRO-1, PO-PRO-3, LO-PRO-1, BO-PRO-3, YO-PRO-3, TO-PRO-3, TO-PRO-5 (and fluorophores derived from these compounds, which have a modified linker structure), particularly preferably thiazole orange,
M is a substituted carbon moiety of the CH or CR type (wherein R is hydrogen or an organic radical, preferably a substituted or unsubstituted methyl group, furthermore preferably a substituted or unsubstituted alkyl group, furthermore preferably a substituted or unsubstituted phenyl group), a substituted amino group of the NH or NR type (wherein R is hydrogen or an organic radical, preferably a substituted or unsubstituted methyl group, furthermore preferably a substituted or unsubstituted alkyl group, furthermore preferably a substituted or unsubstituted phenyl group) or an atom of the 5th or 6th main group of the Periodic Table, preferably oxygen or sulfur, particularly preferably a CH group,
A' is a methylene group or a substituted carbon moiety of the CHR or CR2 type (wherein R is hydrogen or an organic radical, preferably a substituted or unsubstituted methyl group, furthermore preferably a substituted or unsubstituted alkyl group, furthermore preferably a substituted or unsubstituted phenyl group), a substituted or unsubstituted amino group or an atom of the 5th or 6th main group of the Periodic Table, preferably sulfur, preferably a methylene group,
n is a natural number from 0-5.

3. The probe as claimed in either or both of claims 1 and 2, **characterized in that** a plurality of units of the general structure (I) or (II) are covalently linked and a probe having multiple labelings is obtained.

4. A process for preparing the probe as claimed in any of claims 1-3 by solid phase synthesis, preferably by automated solid phase synthesis.

5. The use of the probe as claimed in any of claims 1-3 for carrying out nucleic acid assays, preferably for carrying out homogeneous nucleic acid assays.

6. The use of the probe as claimed in any of claims 1-3 for carrying out a PCR reaction, preferably for carrying out a quantitative PCR reaction.

7. The use of the probe as claimed in any of claims 1-3 for carrying out multiplex assays, **characterized in that** different probes are labeled by different fluorescent base surrogates.

8. The probe as claimed in claim 1 or 3, wherein said probe comprises L-omitine to which a fluorescent base surrogate, preferably thiazole orange, is bound.

9. The probe as claimed in claim 1 or 3, wherein said probe comprises D-ornitine to which a fluorescent base surrogate, preferably thiazole orange, is bound.

10. A test kit comprising those reagents which are required for carrying out the assays as claimed in any of claims 5-7.

## Revendications

1. Sonde de PNA de formule générale 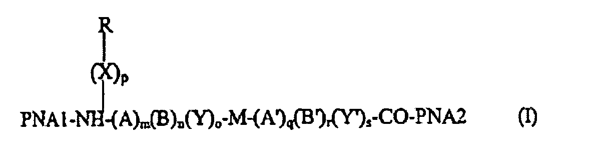 dans laquelle
PNA1 représente un brin d'acide nucléique peptidique de séquence et de longueur quelconques, avantageusement une séquence de 2 à 100 bases, très avantageusement entre 2 et 10 bases,
PNA2 représente un brin d'acide nucléique peptidique de séquence et de longueur quelconques, avantageusement une séquence de 2 à 100 bases, très avantageusement entre 2 et 10 bases,
A est un groupe méthylène ou un motif carboné substitué du type CHR ou CR2 (R représentant l'hydrogène ou un reste organique, avantageusement un groupe méthyle substitué ou non substitué, avantageusement en outre un groupe alkyle substitué ou non substitué, avantageusement en outre un groupe phényle substitué ou non substitué), préférentiellement un groupe méthylène,
B représente un groupe méthylène ou un motif carboné substitué du type CHR ou CR2 (R représentant l'hydrogène ou un reste organique, avantageusement un groupe méthyle substitué ou non substitué, avantageusement en outre un groupe alkyle substitué ou non substitué, avantageusement en outre un groupe phényle substitué ou non substitué), un groupe amino substitué ou non substitué ou un atome du 5^{e} ou du 6^{e} groupe principal du Système Périodique, avantageusement l'oxygène ou le soufre, très avantageusement un groupe méthylène,
C représente un groupe méthylène ou un motif carboné substitué du type CHR ou CR2 (R représentant l'hydrogène ou un reste organique, avantageusement un groupe méthyle substitué ou non substitué, avantageusement en outre un groupe alkyle substitué ou non substitué, avantageusement en outre un groupe phényle substitué ou non substitué), un groupe amino substitué ou non substitué ou un atome du 5^{e} ou du 6^{e} groupe principal du Système Périodique, avantageusement l'oxygène ou le soufre, très avantageusement un groupe méthylène,
m est un nombre naturel de 1 à 5, avantageusement le nombre 1,
n est un nombre naturel de 1 à 5, avantageusement le nombre 1,
o est un nombre naturel de 1 à 5, avantageusement le nombre 1,
X représente un groupe méthylène ou un motif carboné substitué du type CHR ou CR2 (R représentant l'hydrogène ou un reste organique, avantageusement un groupe méthyle substitué ou non substitué, avantageusement en outre un groupe alkyle substitué ou non substitué, avantageusement en outre un groupe phényle substitué ou non substitué), un groupe amino substitué ou non substitué du type NH ou NR (R représentant l'hydrogène ou un reste organique, avantageusement un groupe méthyle substitué ou non substitué, avantageusement en outre un groupe alkyle substitué ou non substitué, avantageusement en outre un groupe phényle substitué ou non substitué) ou un atome du 5^{e} ou du 6^{e} groupe principal du Système Périodique, avantageusement l'oxygène ou le soufre, très avantageusement un groupe NH,
p est un nombre naturel de 0 à 10, avantageusement un nombre de 1 à 6,
R est un substitut de base universel fluorescent, **caractérisé en ce qu'**il est fluorescent et susceptible d'intercalation dans un ADN, avantageusement un colorant dérivé de cyanine, avantageusement en outre l'éthidium, avantageusement en outre un anthracène substitué, avantageusement en outre un colorant dérivé d'acridine, avantageusement en outre un colorant choisi parmi les colorants PO-PRO-1, BO-PRO-1, YO-PRO-1, TO-PRO-1, JO-PRO-1, PO-PRO-1, PO-PRO-3, LO-PRO-1, BO-PRO-3, YO-PRO-3, TO-PRO-3, TO-PRO-5 commercialisés par la firme Molecular Probes (ainsi que des fluorophores à structure de linker modifiée dérivés de ces composés), très avantageusement l'orange de thiazole,
M est un motif carboné substitué du type CH ou CR (R représentant l'hydrogène ou un reste organique, avantageusement un groupe méthyle substitué ou non substitué, avantageusement en outre un groupe alkyle substitué ou non substitué, avantageusement en outre un groupe phényle substitué ou non substitué), un groupe amino substitué du type NH ou NR (R représentant l'hydrogène ou un reste organique, avantageusement un groupe méthyle substitué ou non substitué, avantageusement en outre un groupe alkyle substitué ou non substitué, avantageusement en outre un groupe phényle substitué ou non substitué) ou un atome du 5^{e} ou du 6^{e} groupe principal du Système Périodique, avantageusement l'oxygène ou le soufre, très avantageusement un groupe CH,
A' représente un groupe méthylène ou un motif carboné substitué du type CHR ou CR2 (R représentant l'hydrogène ou un reste organique, avantageusement un groupe méthyle substitué ou non substitué, avantageusement en outre un groupe alkyle substitué ou non substitué, avantageusement en outre un groupe phényle substitué ou non substitué), un groupe amino substitué ou non substitué ou un atome du 5^{e} ou du 6^{e} groupe principal du Système Périodique, avantageusement l'oxygène ou le soufre, très avantageusement un groupe méthylène,
B' représente un groupe méthylène ou un motif carboné substitué du type CHR ou CR2 (R représentant l'hydrogène ou un reste organique, avantageusement un groupe méthyle substitué ou non substitué, avantageusement en outre un groupe alkyle substitué ou non substitué, avantageusement en outre un groupe phényle substitué ou non substitué), un groupe amino substitué ou non substitué ou un atome du 5^{e} ou du 6^{e} groupe principal du Système Périodique, avantageusement l'oxygène ou le soufre, très avantageusement un groupe méthylène,
Y' est un groupe méthylène ou un motif carboné substitué du type CHR ou CR2 (R représentant l'hydrogène ou un reste organique, avantageusement un groupe méthyle substitué ou non substitué, avantageusement en outre un groupe alkyle substitué ou non substitué, avantageusement en outre un groupe phényle substitué ou non substitué), très avantageusement un groupe méthylène,
q est un nombre entier de 0 à 5, avantageusement le nombre 0
r est un nombre entier de 0 à 5, avantageusement le nombre 0,
s est un nombre entier de 0 à 5, avantageusement le nombre 0.

2. Sonde de formule générale 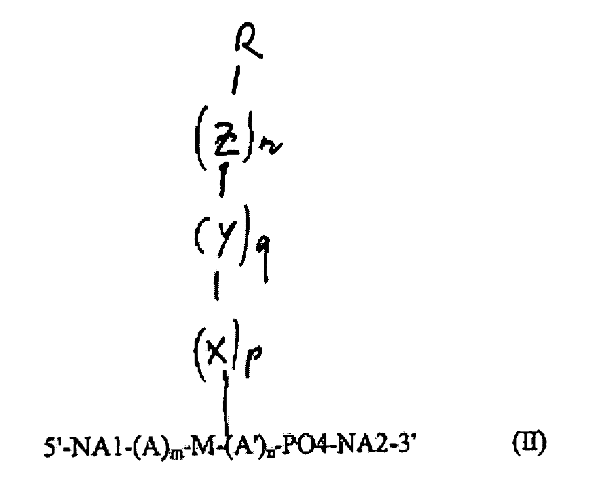 dans laquelle
5'-NAl représente un acide nucléique ou un analogue d'acide nucléique de séquence et de longueur quelconques, qui a pour extrémité un groupe phosphate 3'-terminal, avantageusement une séquence d'acide nucléique d'une longueur de 3 à 100 bases, de façon particulièrement appréciée une séquence d'acide nucléique d'une longueur de 3 à 20 bases et de façon tout particulièrement appréciée une séquence d'acide nucléique d'une longueur de 3 à 15 bases,
NA2-3' représente un acide nucléique ou un analogue d'acide nucléique de séquence et de longueur quelconques, avantageusement une séquence d'acide nucléique d'une longueur de 3 à 100 bases, de façon particulièrement appréciée une séquence d'acide nucléique d'une longueur de 3 à 20 bases et de façon tout particulièrement appréciée une séquence d'acide nucléique d'une longueur de 3 à 15 bases,
A est un groupe méthylène ou un motif carboné substitué du type CHR ou CR2 (R représentant l'hydrogène ou un reste organique, avantageusement un groupe méthyle substitué ou non substitué, avantageusement en outre un groupe alkyle substitué ou non substitué, avantageusement en outre un groupe phényle substitué ou non substitué), préférentiellement un groupe méthylène,
m est un nombre naturel de 0 à 5,
X représente un groupe méthylène ou un motif carboné du type CHR ou CR2 (R représentant l'hydrogène ou un reste organique, avantageusement un groupe méthyle substitué ou non substitué, avantageusement en outre un groupe alkyle substitué ou non substitué, avantageusement en outre un groupe phényle substitué ou non substitué), un groupe amino substitué ou non substitué du type NH ou NR (R représentant l'hydrogène ou un reste organique, avantageusement un groupe méthyle substitué ou non substitué, avantageusement en outre un groupe alkyle substitué ou non substitué, avantageusement en outre un groupe phényle substitué ou non substitué) ou un atome du 5^{e} ou du 6^{e} groupe principal du Système Périodique, avantageusement l'oxygène ou le soufre, très avantageusement un groupe méthylène,
Y représente un groupe méthylène ou un motif carboné du type CHR ou CR2 (R représentant l'hydrogène ou un reste organique, avantageusement un groupe méthyle substitué ou non substitué, avantageusement en outre un groupe alkyle substitué ou non substitué, avantageusement en outre un groupe phényle substitué ou non substitué), un groupe amino substitué ou non substitué du type NH ou NR (R représentant l'hydrogène ou un reste organique, avantageusement un groupe méthyle substitué ou non substitué, avantageusement en outre un groupe alkyle substitué ou non substitué, avantageusement en outre un groupe phényle substitué ou non substitué) ou un atome du 5^{e} ou du 6^{e} groupe principal du Système Périodique, avantageusement l'oxygène ou le soufre, très avantageusement un groupe NH,
Z représente un groupe méthylène ou un motif carboné substitué du type CHR ou CR2 (R représentant l'hydrogène ou un reste organique, avantageusement un groupe méthyle substitué ou non substitué, avantageusement en outre un groupe alkyle substitué ou non substitué, avantageusement en outre un groupe phényle substitué ou non substitué), un groupe amino substitué ou non substitué du type NH ou NR (R représentant l'hydrogène ou un reste organique, avantageusement un groupe méthyle substitué ou non substitué, avantageusement en outre un groupe alkyle substitué ou non substitué, avantageusement en outre un groupe phényle substitué ou non substitué) ou un atome du 5^{e} ou du 6^{e} groupe principal du Système Périodique, avantageusement l'oxygène ou le soufre, très avantageusement un groupe méthylène,
p représente un nombre naturel de 0 à 10, avantageusement un nombre de 1 à 6, très avantageusement le nombre 1,
q représente un nombre naturel de 0 à 10, avantageusement un nombre de 1 à 6, très avantageusement le nombre 1,
r représente un nombre naturel de 0 à 10, avantageusement un nombre de 1 à 6,
R est un substitut de base universel fluorescent, **caractérisé en ce qu'**il est fluorescent et susceptible d'intercalation dans un ADN, avantageusement un colorant dérivé de cyanine, avantageusement en outre l'éthidium, avantageusement en outre un anthracène substitué, avantageusement en outre un colorant dérivé d'acridine, avantageusement en outre un colorant choisi parmi les colorants PO-PRO-1, BO-PRO-1, YO-PRO-1, TO-PRO-1, JO-PRO-1, PO-PRO-1, PO-PRO-3, LO-PRO-1, BO-PRO-3, YO-PRO-3, TO-PRO-3, TO-PRO-5 commercialisés par la firme Molecular Probes (ainsi que des fluorophores à structure de linker modifiée dérivés de ces composés), très avantageusement l'orange de thiazole,
M est un motif carboné substitué du type CH ou CR (R représentant l'hydrogène ou un reste organique, avantageusement un groupe méthyle substitué ou non substitué, avantageusement en outre un groupe alkyle substitué ou non substitué, avantageusement en outre un groupe phényle substitué ou non substitué), un groupe amino substitué ou non substitué du type NH ou NR (R représentant l'hydrogène ou un reste organique, avantageusement un groupe méthyle substitué ou non substitué, avantageusement en outre un groupe alkyle substitué ou non substitué, avantageusement en outre un groupe phényle substitué ou non substitué) ou un atome du 5^{e} ou du 6^{e} groupe principal du Système Périodique, avantageusement l'oxygène ou le soufre, très avantageusement un groupe CH,
A' représente un groupe méthylène ou un motif carboné substitué du type CHR ou CR2 (R représentant l'hydrogène ou un reste organique, avantageusement un groupe méthyle substitué ou non substitué, avantageusement en outre un groupe alkyle substitué ou non substitué, avantageusement en outre un groupe phényle substitué ou non substitué), un groupe amino substitué ou non substitué ou un atome du 5^{e} ou du 6^{e} groupe principal du Système Périodique, avantageusement le soufre, très avantageusement un groupe méthylène,
n est un nombre naturel de 0 à 5.

3. Sonde suivant l'une des revendications 1 et/ou 2, **caractérisée en ce que** plusieurs motifs de formule générale (I) ou (II) sont liés par covalence et une sonde à marquages multiples est obtenue.

4. Procédé de production d'une sonde suivant l'une des revendications 1 à 3 par synthèse en phase solide, avantageusement par synthèse automatisée en phase solide.

5. Utilisation d'une sonde suivant l'une des revendications 1 à 3 pour la conduite d'analyses d'acides nucléiques, avantageusement pour la conduite d'analyses homogènes d'acides nucléiques.

6. Utilisation de la sonde suivant l'une des revendications 1 à 3 pour la conduite d'une réaction PCR, avantageusement pour la conduite d'une réaction PCR quantitative.

7. Utilisation de la sonde suivant l'une des revendications 1 à 3 pour la conduite d'analyses multiplex, **caractérisée en ce que** diverses sondes sont marquées par divers substituts de bases fluorescents.

8. Sonde suivant la revendication 1 ou 3, laquelle sonde contient de la L-ornithine à laquelle est lié un substitut de base fluorescent, avantageusement l'orange de thiazole.

9. Sonde suivant la revendication 1 ou 3, laquelle sonde contient de la D-ornithine à laquelle est lié un substitut de base fluorescent, avantageusement l'orange de thiazole.

10. Kit pour tests, qui contient les réactifs que nécessite la conduite des analyses suivant l'une des revendications 5 à 7.
